# EUROPÄISCHE PATENTSCHRIFT

(11) **EP 2 035 419 B1**
(45) Veröffentlichungstag und Bekanntmachung des Hinweises auf die Patenterteilung: **22.06.2016**
(21) Anmeldenummer: 07725586.7
(22) Anmeldetag: 25.05.2007
(51) Int. Cl.: C07D 471/04, A61K 31/519, A61K 31/5025, A61P 5/42, A61P 9/00

(54) **5-ARYL-SUBSTITUIERTE DIHYDROPYRIDOPYRIMIDINE UND DIHYDROPYRIDAZINE UND IHRE VERWENDUNG ALS MINERALKORTICOIDANATGONISTEN**
5-ARYL-SUBSTITUTED DIHYDROPYRIDOPYRIMIDINES AND DIHYDROPYRIDAZINES AND USE THEREOF AS MINERAL CORTICOID ANTAGONISTS
DIHYDROPYRIDOPYRIMIDINES ET DIHYDROPYRIDAZINES À SUBSTITUTION 5-ARYLE ET LEUR UTILISATION COMME ANTAGONISTES DES CORTICOÏDES MINÉRAUX

(30) Priorität: 07.06.2006 DE 102006026583
(43) Veröffentlichungstag der Anmeldung: 18.03.2009
(73) Patentinhaber: Bayer Intellectual Property GmbH, 40789 Monheim (DE)
(72) Erfinder: FIGUEROA PEREZ, Santiago, 51373 Leverkusen (DE); KOLKHOF, Peter, 42113 Wuppertal (DE); BÄRFACKER, Lars, 46047 Oberhausen (DE); FLAMME, Ingo, 51580 Reichshof (DE); SCHLEMMER, Karl-Heinz, 42113 Wuppertal (DE); KUHL, Alexander, 58093 Hagen (DE); GROSSER, Rolf, 51375 Leverkusen (DE); MÜNTER, Klaus, 42489 Wülfrath (DE); KNORR, Andreas, 40699 Erkrath (DE)
(74) Vertreter: BIP Patents
(86) Internationale Anmeldenummer: PCT/EP2007/004690
(87) Internationale Veröffentlichungsnummer: WO 2007/140894

(56) Entgegenhaltungen:
- EP-A1- 0 180 834
- WO-A-2005/087740

## Beschreibung

Die vorliegende Anmeldung betrifft neue, Aryl-substituierte hetero-bicyclische Verbindungen, Verfahren zu ihrer Herstellung, ihre Verwendung zur Behandlung und/oder Prophylaxe von Krankheiten sowie ihre Verwendung zur Herstellung von Arzneimitteln zur Behandlung und/oder Prophylaxe von Krankheiten, insbesondere von kardiovaskulären Erkrankungen.

Aldosteron spielt eine Schlüsselrolle in der Aufrechterhaltung der Flüssigkeits- und Elektrolythomöostase, indem es im Epithel des distalen Nephrons die Natriumretention und Kaliumsekretion fördert, was zur Konstanthaltung des Extrazellulärvolumens und damit zur Blutdruckregulation beiträgt. Daneben entfaltet Aldosteron direkte Effekte auf die Struktur und Funktion des Herz- und Gefäßsystems, wobei die dafür zugrunde liegenden Mechanismen noch nicht erschöpfend geklärt sind [R.E. Booth, J.P. Johnson, J.D. Stockand, Adv. Physiol. Educ. 26 (1), 8-20 (2002)].

Aldosteron ist ein Steroidhormon, das in der Nebennierenrinde gebildet wird. Seine Produktion wird indirekt ganz wesentlich in Abhängigkeit von der Nierendurchblutung reguliert. Jede Abnahme der Nierendurchblutung führt in der Niere zu einer Ausschüttung des Enzyms Renin in den Blutkreislauf. Dieses wiederum aktiviert die Bildung von Angiotensin II, das einerseits verengend auf die arteriellen Blutgefäße wirkt, andererseits aber auch die Bildung von Aldosteron in der Nebennierenrinde stimuliert. Somit fungiert die Niere als Blutdruck- und damit indirekter Volumen-Sensor im Blutkreislauf und wirkt über das Renin-Angiotensin-Aldosteron-System kritischen Volumenverlusten entgegen, indem einerseits der Blutdruck gesteigert wird (Angiotensin II-Wirkung), andererseits durch verstärkte Rückresorption von Natrium und Wasser in der Niere der Füllungszustand des Gefäßsystems wieder ausgeglichen wird (Aldosteron-Wirkung).

Dieses Regelsystem kann in vielfältiger Weise krankhaft gestört sein. So führt eine chronische Minderdurchblutung der Nieren (z.B. infolge einer Herzinsuffizienz und des hierdurch verursachten Blutrückstaus im Venensystem) zu einer chronisch überhöhten Ausschüttung von Aldosteron. Diese wiederum zieht eine Expansion des Blutvolumens nach sich und verstärkt hiermit die Herzschwäche durch ein Volumenüberangebot an das Herz. Eine Stauung von Blut in den Lungen mit Atemnot und Ödembildung in den Extremitäten sowie Aszites und Pleuraergüsse können die Folge sein; die Nierendurchblutung sinkt weiter ab. Überdies führt die übersteigerte Aldosteron-Wirkung zu einer Minderung der Kalium-Konzentration im Blut und in der Extrazellulärflüssigkeit. In ohnehin vorgeschädigten Herzmuskeln kann die Unterschreitung eines kritischen Mindestwertes tödlich endende Herzrhythmusstörungen auslösen. Hierin dürfte eine der Hauptursachen des häufig bei Patienten mit Herzinsuffizienz eintretenden *plötzlichen Herztodes* zu suchen sein.

Zusätzlich wird Aldosteron auch für eine Reihe der typischerweise bei Herzinsuffizienten zu beobachtenden Umbauprozesse des Herzmuskels verantwortlich gemacht. Somit ist der *Hyperaldosteronismus* eine entscheidende Komponente in der Pathogenese und Prognose der Herzinsuffizienz, die ursprünglich durch unterschiedliche Schädigungen, wie z.B. einen Herzinfarkt, eine Herzmuskelentzündung oder Bluthochdruck, ausgelöst werden kann. Diese Annahme wird durch die Tatsache erhärtet, dass in umfangreichen klinischen Studien in Patientenkollektiven mit chronischer Herzinsuffizienz bzw. nach akutem Myokardinfarkt durch Anwendung von Aldosteron-Antagonisten die Gesamtmortalität deutlich gesenkt wurde [B. Pitt, F. Zannad, W.J. Remme et al., N. Engl. J. Med. 341, 709-717 (1999); B. Pitt, W. Remme, F. Zannad et al., N. Engl. J. Med. 348, 1309-1321 (2003)]. Dies konnte unter anderem durch eine Senkung der Inzidenz des plötzlichen Herztodes erreicht werden.

Neueren Studien zufolge findet man auch bei einem nicht unerheblichen Teil der Patienten, die unter einer essentiellen Hypertonie leiden, eine sogenannte normokaliämische Variante des primären Hyperaldosteronismus [Prävalenz bis 11% aller Hypertoniker: L. Seiler und M. Reincke, Der Aldosteron-Renin-Quotient bei sekundärer Hypertonie, Herz 28, 686-691 (2003)]. Als beste Diagnostikmethode dient beim normokaliämischen Hyperaldosteronismus der Aldosteron/Renin-Quotient der entsprechenden Plasmakonzentrationen, so dass auch relative Aldosteron-Erhöhungen in Bezug auf die Renin-Plasmakonzentrationen diagnostizierbar und letztlich therapierbar werden. Deshalb ist ein im Zusammenhang mit einer essentiellen Hypertonie diagnostizierter Hyperaldosteronismus ein Ansatzpunkt für eine kausale und prophylaktisch sinnvolle Therapie.

Weitaus seltener als die oben aufgeführten Formen des Hyperaldosteronismus sind solche Krankheitsbilder, bei denen die Störung entweder in den Hormon-produzierenden Zellen der Nebenniere selbst zu finden ist oder deren Anzahl oder Masse durch eine Hyperplasie oder Wucherung vermehrt ist. Adenome oder diffuse Hyperplasien der Nebennierenrinde sind die häufigste Ursache des auch als *Conn-Syndrom* bezeichneten primären Hyperaldosteronismus, dessen Leitsymptome Hypertonie und hypokaliämische Alkalose sind. Auch hier steht neben der chirurgischen Entfernung des krankhaften Gewebes die medikamentöse Therapie mit Aldosteron-Antagonisten im Vordergrund [H.A. Kühn und J. Schirmeister (Hrsg.), Innere Medizin, 4. Aufl., Springer Verlag, Berlin, 1982].

Ein anderes typischerweise mit Erhöhung der Aldosteron-Konzentration im Plasma einhergehendes Krankheitsbild ist die fortgeschrittene Leberzirrhose. Die Ursache der Aldosteronerhöhung liegt hier vorwiegend im infolge der Leberfunktionsstörung eingeschränkten Abbau des Aldosterons. Volumenüberlastung, Ödeme und Hypokaliämie sind die typischen Folgen, die in der klinischen Praxis durch Aldosteron-Antagonisten erfolgreich gelindert werden können.

Die Wirkungen von Aldosteron werden über den in den Zielzellen intrazellulär lokalisierten Mineralokorticoid-Rezeptor vermittelt. Die bislang verfügbaren Aldosteron-Antagonisten besitzen wie Aldosteron selbst eine Steroid-Grundstruktur. Begrenzt wird die Anwendbarkeit derartiger steroidaler Antagonisten durch ihre Wechselwirkungen mit den Rezeptoren anderer Steroidhormone, die zum Teil zu erheblichen Nebenwirkungen wie Gynäkomastie und Impotenz und zum Abbrechen der Therapie führen [M.A. Zaman, S. Oparil, D.A. Calhoun, Nature Rev. Drug Disc. 1, 621-636 (2002)].

Die Anwendung wirkstarker, nicht-steroidaler und für den Mineralokorticoid-Rezeptor selektiver Antagonisten bietet die Möglichkeit, dieses Nebenwirkungsprofil zu umgehen und dadurch einen deutlichen Therapievorteil zu erzielen.

Aufgabe der vorliegenden Erfindung ist die Bereitstellung neuer Verbindungen, die als selektive Mineralokorticoid-Rezeptor-Antagonisten für die Behandlung von Erkrankungen, insbesondere von kardiovaskulären Erkrankungen, eingesetzt werden können.

In EP 0 133 530-A, EP 0 173 933-A, EP 0 189 898-A und EP 0 234 516-A werden 4-Aryl-substituierte 1,4-Dihydro-1,6-naphthyridine bzw. -naphthyridinone mit Calcium-antagonistischer Wirkung zur Behandlung von Gefäßerkrankungen offenbart. Weiterhin werden 1,4-Dihydro-1,6-naphthyridin-Derivate in WO 02/10164 als Kaliumkanal-Öffner zur Behandlung unterschiedlicher, vor allem urologischer Erkrankungen beansprucht. 4-Fluorenonyl- bzw. 4-Chromenonyl-1,4-dihydropyridin-Derivate als Mineralokorticoid-Rezeptor-Antagonisten werden in WO 2005/087740 und WO 2007/009670 beschrieben. In WO 2006/066011 werden 4-Aryl-3-cyano-1,4-dihydropyridin-5-carbonsäureester und -amide als zum Teil duale Modulatoren von Steroidhormon-Rezeptoren und des L-Typ-Calciumkanals offenbart, und in WO 2005/097118 werden Verbindungen mit 4-Aryl-1,4-dihydropyridin-Kernstruktur als Aldosteron-Rezeptor-Antagonisten beansprucht.

Beschrieben sind Verbindungen der allgemeinen Formel (I) 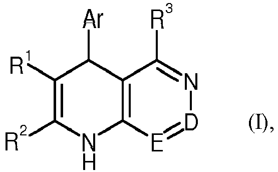 in welcher
- D: für N oder C-R⁴ steht, worin
R⁴ Wasserstoff, (C₁-C₆)-Alkyl, Trifluormethyl, (C₁-C₆)-Alkoxy, Trifluormethoxy, (C₁-C₆)-Alkylthio, Amino, Mono-(C₁-C₆)-alkylamino oder Di-(C₁-C₆)-alkylamino bedeutet,
- E: für N oder C-R⁵ steht, worin
R⁵ Wasserstoff oder (C₁-C₄)-Alkyl bedeutet,
wobei entweder D für C-R⁴ und E für N oder D für N und E für C-R⁵ stehen,
- Ar: für eine Gruppe der Formel 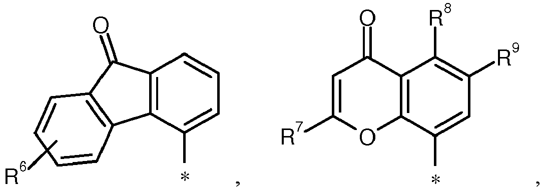 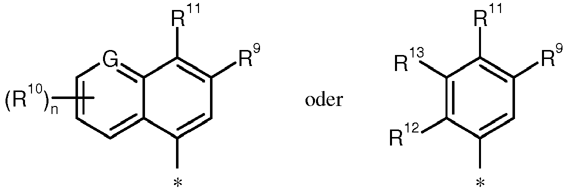 steht, worin
* die Verknüpfungsstelle bedeutet,
R⁶ Wasserstoff oder Halogen bedeutet,
R⁷ Methyl oder Ethyl bedeutet,
R⁸ Wasserstoff, Fluor, Chlor, Cyano, Nitro, Trifluormethyl oder (C₁-C₄)-Alkyl bedeutet,
R⁹ Wasserstoff oder Fluor bedeutet,
R¹⁰ Halogen, (C₁-C₄)-Alkyl, Trifluormethyl, (C₁-C₄)-Alkoxy oder Trifluormethoxy bedeutet,
R¹¹ Cyano oder Nitro bedeutet,
R¹² Wasserstoff, Halogen, (C₁-C₄)-Alkyl, (C₁-C₄)-Alkoxy, (C₁-C₄)-Alkylthio oder Di-(C₁-C₄)-alkylamino, wobei die Alkylgruppe in den genannten (C₁-C₄)-Alkyl-, (C₁-C₄)-Alkoxy- und (C₁-C₄)-Alkylthio-Resten jeweils bis zu dreifach mit Fluor substituiert sein kann,
oder
Phenyl, welches mit Halogen, (C₁-C₄)-Alkyl oder Trifluormethyl substituiert sein kann, bedeutet,
R¹³ Wasserstoff, Halogen oder (C₁-C₄)-Alkyl bedeutet,
G CH, C-R¹⁰ oder N bedeutet
und
n die Zahl 0, 1 oder 2 bedeutet,
wobei für den Fall, dass der Substituent R¹⁰ mehrfach auftritt, seine Bedeutungen gleich oder verschieden sein können,
- R¹: für Cyano, Nitro oder eine Gruppe der Formel -C(=O)-R¹⁴ oder -C(=O)-O-R¹⁵ steht, worin
R¹⁴ (C₁-C₆)-Alkyl, das mit (C₃-C₇)-Cycloalkyl oder ein- bis dreifach mit Fluor substituiert sein kann, oder Phenyl, das mit Halogen, Cyano, (C₁-C₄)-Alkyl, Trifluormethyl, (C₁-C₄)-Alkoxy oder Trifluormethoxy substituiert sein kann, oder (C₃-C₇)-Cycloalkyl bedeutet
und
R¹⁵ (C₁-C₆)-Alkyl, das mit (C₃-C₇)-Cycloalkyl oder ein- bis dreifach mit Fluor substituiert sein kann, oder (C₃-C₇)-Cycloalkyl bedeutet,
- R²: für (C₁-C₄)-Alkyl, Trifluormethyl, Cyclopropyl, Cyclobutyl, (C₁-C₄)-Alkoxy oder (C₁-C₄)-Alkylthio steht
und
- R³: für (C₁-C₆)-Alkyl, (C₁-C₆)-Alkoxy, Trifluormethoxy, (C₁-C₆)-Alkylthio, Amino, Mono-(C₁-C₆)-alkylamino oder eine Gruppe der Formel -O-SO₂-R¹⁶ steht,
wobei die genannten (C₁-C₆)-Alkyl-, (C₁-C₆)-Alkoxy- und (C₁-C₆)-Alkylthio-Reste jeweils mit (C₃-C₇)-Cycloalkyl substituiert sein können
und
R¹⁶ (C₁-C₆)-Alkyl, Trifluormethyl, (C₃-C₇)-Cycloalkyl, Phenyl oder 5- oder 6-gliedriges Heteroaryl mit bis zu zwei Heteroatomen aus der Reihe N, O und/oder S bedeutet,
wobei Phenyl und Heteroaryl ihrerseits jeweils ein- oder zweifach, gleich oder verschieden, mit Halogen, Cyano, Nitro, (C₁-C₄)-Alkyl, Trifluormethyl, (C₁-C₄)-Alkoxy und/oder Trifluormethoxy substituiert sein können,
sowie ihre Salze, Solvate und Solvate der Salze.

Erfindungsgemäße Verbindungen sind die Verbindungen der Formel (I) gemäss Anspruch 1 und deren Salze, Solvate und Solvate der Salze, die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die Erfindung umfasst deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegende Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin und N-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Außerdem umfasst die vorliegende Erfindung auch Prodrugs der erfindungsgemäßen Verbindungen. Der Begriff "Prodrugs" umfasst Verbindungen, welche selbst biologisch aktiv oder inaktiv sein können, jedoch während ihrer Verweilzeit im Körper zu erfindungsgemäßen Verbindungen umgesetzt werden (beispielsweise metabolisch oder hydrolytisch).

Im Rahmen der vorliegenden Erfindung haben die Substituenten, soweit nicht anders spezifiziert, die folgende Bedeutung:
(C₁-C₆)-Alkyl, (C₁-C₄)-Alkyl und (C₁-C₃)-Alkyl stehen im Rahmen der Erfindung für einen geradkettigen oder verzweigten Alkylrest mit 1 bis 6, 1 bis 4 bzw. 1 bis 3 Kohlenstoffatomen. Bevorzugt ist ein geradkettiger oder verzweigter Alkylrest mit 1 bis 4, besonders bevorzugt mit 1 bis 3 Kohlenstoffatomen. Beispielhaft und vorzugsweise seien genannt: Methyl, Ethyl, n-Propyl, Isopropyl, n-Butyl, iso-Butyl, sec.-Butyl, tert.-Butyl, 1-Ethylpropyl, n-Pentyl, iso-Pentyl und n-Hexyl.

(C₃-C₇)-Cycloalkyl, (C₃-C₆)-Cycloalkyl und C₃-C₅)-Cycloalkyl stehen im Rahmen der Erfindung für eine monocyclische, gesättigte Cycloalkylgruppe mit 3 bis 7, 3 bis 6 bzw. 3 bis 5 Kohlenstoffatomen. Bevorzugt ist ein Cycloalkylrest mit 3 bis 6, besonders bevorzugt mit 3 bis 5 Kohlenstoffatomen. Beispielhaft und vorzugsweise seien genannt: Cyclopropyl, Cyclobutyl, Cyclopentyl, Cyclohexyl und Cycloheptyl.

(C₁-C₆)-Alkoxy, (C₁-C₄)-Alkoxy und (C₁-C₃)-Alkoxy stehen im Rahmen der Erfindung für einen geradkettigen oder verzweigten Alkoxyrest mit 1 bis 6, 1 bis 4 bzw. 1 bis 3 Kohlenstoffatomen. Bevorzugt ist ein geradkettiger oder verzweigter Alkoxyrest mit 1 bis 4, besonders bevorzugt mit 1 bis 3 Kohlenstoffatomen. Beispielhaft und vorzugsweise seien genannt: Methoxy, Ethoxy, n-Propoxy, Isopropoxy, n-Butoxy, tert.-Butoxy, n-Pentoxy und n-Hexoxy.

(C₁-C₆)-Alkylthio und (C₁-C₄)-Alkylthio stehen im Rahmen der Erfindung für einen geradkettigen oder verzweigten Alkylthiorest mit 1 bis 6 bzw. 1 bis 4 Kohlenstoffatomen. Bevorzugt ist ein geradkettiger oder verzweigter Alkylthiorest mit 1 bis 4 Kohlenstoffatomen. Beispielhaft und vorzugsweise seien genannt: Methylthio, Ethylthio, n-Propylthio, Isopropylthio, n-Butylthio, tert.-Butylthio, n-Pentylthio und n-Hexylthio.

Mono-(C₁-C₆)-alkylamino und Mono-(C₁-C₄)-alkylamino stehen im Rahmen der Erfindung für eine Amino-Gruppe mit einem geradkettigen oder verzweigten Alkylsubstituenten, der 1 bis 6 bzw. 1 bis 4 Kohlenstoffatome aufweist. Bevorzugt ist ein geradkettiger oder verzweigter Monoalkylamino-Rest mit 1 bis 4 Kohlenstoffatomen. Beispielhaft und vorzugsweise seien genannt: Methylamino, Ethylamino, n-Propylamino, Isopropylamino, n-Butylamino, tert.-Butylamino, n-Pentylamino und n-Hexylamino.

Di-(C₁-C₆)-alkylamino und Di-(C₁-C₄)-alkylamino stehen im Rahmen der Erfindung für eine Amino-Gruppe mit zwei gleichen oder verschiedenen geradkettigen oder verzweigten Alkylsubstituenten, die jeweils 1 bis 6 bzw. 1 bis 4 Kohlenstoffatome aufweisen. Bevorzugt sind geradkettige oder verzweigte Dialkylamino-Reste mit jeweils 1 bis 4 Kohlenstoffatomen. Beispielhaft und vorzugsweise seien genannt: *N,N*-Dimethylamino, *N,N*-Diethylamino, *N*-Ethyl-*N*-methylamino, *N*-Methyl-*N*-n-propylamino, *N*-Isopropyl-*N*-n-propylamino, *N*-tert.-Butyl-*N*-methylamino, *N*-Ethyl-*N*-n-pentylamino und *N*-n-Hexyl-*N*-methylamino.

5- oder 6-gliedriges Heteroaryl steht im Rahmen der Erfindung für einen aromatischen Heterocyclus (Heteroaromaten) mit 5 oder 6 Ringatomen, der ein oder zwei Ring-Heteroatome aus der Reihe N, O und/oder S enthält und über ein Ring-Kohlenstoffatom verknüpft ist. Beispielhaft und vorzugsweise seien genannt: Furyl, Pyrrolyl, Thienyl, Pyrazolyl, Imidazolyl, Thiazolyl, Oxazolyl, Isoxazolyl, Isothiazolyl, Pyridyl, Pyrimidinyl, Pyridazinyl und Pyrazinyl.

Halogen schließt im Rahmen der Erfindung Fluor, Chlor, Brom und Iod ein. Bevorzugt sind Fluor oder Chlor.

Wenn Reste in den erfindungsgemäßen Verbindungen substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Eine Substitution mit ein, zwei oder drei gleichen oder verschiedenen Substituenten ist bevorzugt. Ganz besonders bevorzugt ist die Substitution mit einem Substituenten. Ebenfalls beschrieben sind Verbindungen der Formel (I), in welcher
- D: für N oder C-R⁴ steht, worin
R⁴ Wasserstoff, (C₁-C₄)-Alkyl, (C₁-C₄)-Alkoxy, (C₁-C₄)-Alkylthio, Amino, Mono-(C₁-C₄)-alkylamino oder Di-(C₁-C₄)-alkylamino bedeutet,
- E: für N oder C-R⁵ steht, worin
R⁵ Wasserstoff oder Methyl bedeutet,
wobei entweder D für C-R⁴ und E für N oder D für N und E für C-R⁵ stehen,
- Ar: für eine Gruppe der Formel 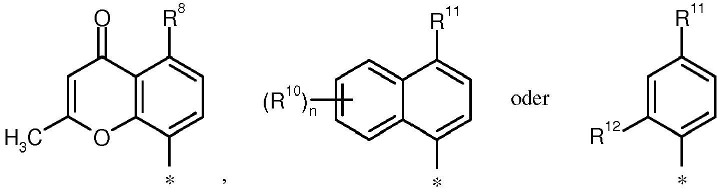 steht, worin
* die Verknüpfungsstelle bedeutet,
R⁸ Wasserstoff, Fluor, Chlor oder Cyano bedeutet,
R¹⁰ Fluor, Chlor, Methyl oder Ethyl bedeutet,
R¹¹ Cyano oder Nitro bedeutet,
R¹² Chlor, Brom, (C₁-C₄)-Alkyl, Trifluormethyl, (C₁-C₄)-Alkoxy, Trifluormethoxy, (C₁-C₄)-Alkylthio oder Trifluoermethylthio bedeutet,
und
n die Zahl 0 oder 1 bedeutet,
- R¹: für Cyano, Acetyl, Trifluoracetyl oder eine Gruppe der Formel -C(=O)-O-R¹⁵ steht, worin
R¹⁵ (C₁-C₄)-Alkyl, das mit (C₃-C₅)-Cycloalkyl oder ein- bis dreifach mit Fluor substituiert sein kann, oder (C₃-C₅)-Cycloalkyl bedeutet,
- R²: für Methyl oder Trifluormethyl steht
und
- R³: für Amino, (C₁-C₄)-Alkoxy, Trifluormethoxy oder eine Gruppe der Formel -O-SO₂-R¹⁶ steht, worin
R¹⁶ (C₁-C₄)-Alkyl, Trifluormethyl, (C₃-C₆)-Cycloalkyl, Phenyl oder Thienyl bedeutet,
wobei Phenyl und Thienyl ihrerseits jeweils ein- oder zweifach, gleich oder verschieden, mit Fluor, Chlor, Cyano, Methyl, Trifluormethyl, Methoxy und/oder Trifluormethoxy substituiert sein können,
sowie ihre Salze, Solvate und Solvate der Salze.

Gegenstand der vorliegenden Erfindung sind Verbindungen der Formel (I), in welcher
- D: für C-R⁴ steht, worin
R⁴ Wasserstoff, Amino, Methoxy oder Methylthio bedeutet,
- E: für N steht,
- Ar: für eine Gruppe der Formel 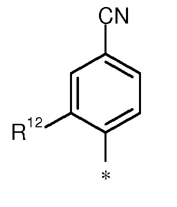 steht, worin
* die Verknüpfungsstelle
und
R¹² Ethyl, Methoxy oder Trifluormethoxy bedeutet,
- R¹: für Cyano, Acetyl, Methoxycarbonyl oder Ethoxycarbonyl steht,
- R²: für Methyl oder Trifluormethyl steht
und
- R³: für Amino, (C₁-C₃)-Alkoxy oder eine Gruppe der Formel -O-SO₂-R¹⁶ steht, worin
R¹⁶ (C₁-C₃)-Alkyl bedeutet,
sowie ihre Salze, Solvate und Solvate der Salze.

Die in den jeweiligen Kombinationen bzw. bevorzugten Kombinationen von Resten im einzelnen angegebenen Reste-Definitionen werden unabhängig von den jeweiligen angegebenen Kombinationen der Reste beliebig auch durch Reste-Definitionen anderer Kombinationen ersetzt. Ganz besonders bevorzugt sind Kombinationen von zwei oder mehreren der oben genannten Vorzugsbereiche.

Weiter beschrieben ist ein Verfahren zur Herstellung der erfindungsgemäßen Verbindungen der Formel (I-A) 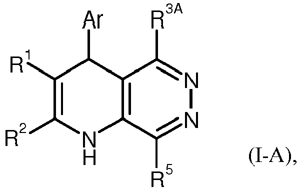 in welcher Ar, R¹, R² und R⁵ jeweils die oben angegebenen Bedeutungen haben
und
- R^{3A}: für (C₁-C₆)-Alkoxy, das mit (C₃-C₇)-Cycloalkyl substituiert sein kann, für Trifluormethoxy oder für eine Gruppe der Formel -O-SO₂-R¹⁶ steht, worin R¹⁶ die oben angegebene Bedeutung hat,
dadurch gekennzeichnet, dass man eine Verbindung der Formel (II) 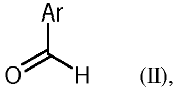 in welcher Ar die oben angegebene Bedeutung hat,
in einem einstufigen (Eintopf-Reaktion) oder zweistufigen Verfahren mit einer Verbindung der Formel (III) 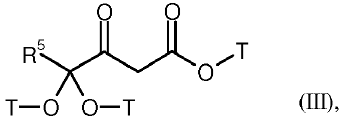 in welcher R⁵ die oben angegebene Bedeutung hat
und
- T: für Methyl oder Ethyl steht,
und einer Verbindung der Formel (IV) 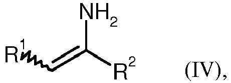 in welcher R¹ und R² die oben angegebenen Bedeutungen haben,
zu einer Verbindung der Formel (V) 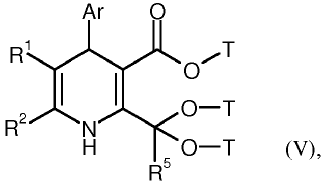 in welcher Ar, R¹, R², R⁵ und T jeweils die oben angegebenen Bedeutungen haben, umsetzt, diese in Gegenwart einer Säure zu einer Verbindung der Formel (VI) 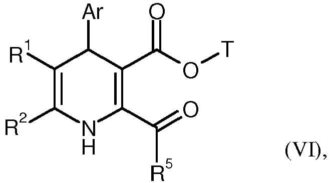 in welcher Ar, R¹, R², R⁵ und T jeweils die oben angegebenen Bedeutungen haben,
hydrolysiert, anschließend mit Hydrazin in Gegenwart einer Säure zu einer Verbindung der Formel (VII) 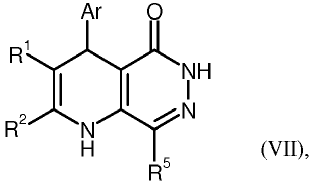 in welcher Ar, R¹, R² und R⁵ jeweils die oben angegebenen Bedeutungen haben,
kondensiert und dann in einem inerten Lösungsmittel gegebenenfalls in Gegenwart einer Base mit einer Verbindung der Formel (VIII) oder einem Trialkyloxonium-Salz der Formel (IX)

R¹⁷-Q (VIII)

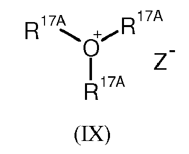

in welchen
- R¹⁷: für (C₁-C₆)-Alkyl, das mit (C₃-C₇)-Cycloalkyl substituiert sein kann, oder für Trifluormethyl steht,
- R^{17A}: für Methyl oder Ethyl steht,
- Q: für eine Abgangsgruppe, wie beispielsweise Halogen, Mesylat, Tosylat oder Triflat, steht und
- Z⁻: für ein nicht-nukleophiles Anion, wie beispielsweise Tetrafluoroborat, steht, zu Verbindungen der Formel (I-A1) 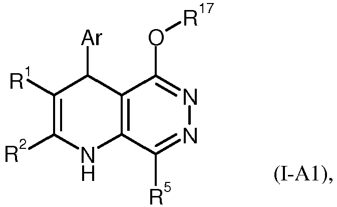
in welcher Ar, R¹, R², R⁵ und R¹⁷ jeweils die oben angegebenen Bedeutungen haben,
alkyliert
oder die Verbindungen der Formel (VII) in einem inerten Lösungsmittel in Gegenwart einer Base mit einer Verbindung der Formel (X) 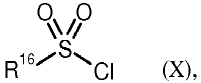 in welcher R¹⁶ die oben angegebene Bedeutung hat,
zu Verbindungen der Formel (I-A2) 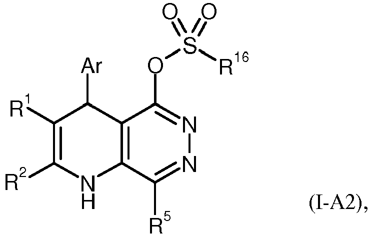 in welcher Ar, R¹, R², R⁵ und R¹⁶ jeweils die oben angegebenen Bedeutungen haben,
umsetzt
und gegebenenfalls die resultierenden Verbindungen der Formel (I-A1) bzw. (I-A2) nach dem Fachmann bekannten Methoden in ihre Enantiomere und/oder Diastereomere trennt und/oder mit den entsprechenden (i) Lösungsmitten und/oder (ii) Basen oder Säuren in ihre Solvate, Salze und/oder Solvate der Salze überführt.

Der Verfahrensschritt (II) + (III) + (IV) → (V) wird im Allgemeinen in einem inerten Lösungsmittel in einem Temperaturbereich von +20°C bis zum Siedepunkt des Lösungsmittels bei Normaldruck durchgeführt.

Als inerte Lösungsmittel eignen sich hierfür beispielsweise Alkohole wie Methanol, Ethanol, n-Propanol, Isopropanol, n-Butanol oder *tert*.-Butanol, Halogenkohlenwasserstoffe wie Dichlormethan, Trichlormethan, Tetrachlormethan, Trichlorethan oder 1,2-Dichlorethan, oder andere Lösungsmittel wie Acetonitril, Tetrahydrofuran, Dioxan, 1,2-Dimethoxyethan, Hexan, Benzol, Toluol, Xylol, Chlorbenzol, Pyridin oder Eisessig. Bevorzugt werden die Umsetzungen in Dichlormethan, Toluol, Ethanol oder Isopropanol bei der jeweiligen Rückfluss-Temperatur unter Normaldruck durchgeführt.

Der Verfahrensschritt (II) + (III) + (IV) → (V) kann gegebenenfalls vorteilhaft in Gegenwart einer Säure, einer Säure/Base-Kombination und/oder eines wasserentziehenden Mittels, wie beispielsweise Molekularsieb, erfolgen. Als Säuren eignen sich beispielsweise Essigsäure, Trifluoressigsäure, Methansulfonsäure oder p-Toluolsulfonsäure; als Basen sind insbesondere Piperidin oder Pyridin geeignet [vgl. nachfolgendes Reaktionsschema 8; zur Synthese von 1,4-Dihydropyridinen vgl. auch D.M. Stout, A.I. Meyers, Chem. Rev. 1982, 82, 223-243; H. Meier et al., Liebigs Ann. Chem. 1977, 1888; H. Meier et al., ibid. 1977, 1895; H. Meier et al., ibid. 1976, 1762; F. Bossert et al., Angew. Chem. 1981, 93, 755].

Der Verfahrensschritt (V) → (VI) wird zweckmäßigerweise in Wasser in Verbindung mit einem wassermischbaren, inerten organischen Lösungsmittel wie Aceton, Tetrahydrofuran, Dioxan oder Essigsäure durchgeführt; bevorzugt wird Aceton eingesetzt. Als Säuren eignen sich für diese Hydrolyse verdünnte wässrige Lösungen von Mineralsäuren, wie beispielsweise Salzsäure, Bromwasserstoffsäure, Schwefelsäure, Salpetersäure oder Phosphorsäure, oder von organischen Säuren wie Essigsäure, Trifluoressigsäure, Methansulfonsäure oder Trifluormethansulfonsäure; bevorzugt wird Salzsäure verwendet.

Die Umsetzung (V) → (VI) erfolgt im Allgemeinen in einem Temperaturbereich von 0°C bis +50°C. Die Reaktion kann bei normalem, erhöhtem oder bei vermindertem Druck durchgeführt werden (z.B. von 0.5 bis 5 bar). Im Allgemeinen arbeitet man bei Normaldruck.

Inerte Lösungsmittel für den Verfahrensschritt (VI) → (VII) sind beispielsweise Alkohole wie Methanol, Ethanol, n-Propanol, Isopropanol, n-Butanol oder *tert*.-Butanol, Ether wie Diethylether, Methyl-*tert*.-butylether, Dioxan, Tetrahydrofuran, Glykoldimethylether oder Diethylenglykoldimethylether, Kohlenwasserstoffe wie Benzol, Toluol oder Xylol, Halogenkohlenwasserstoffe wie Dichlormethan, Trichlormethan, Tetrachlormethan, 1,2-Dichlorethan, Trichlorethan, Tetrachlorethan, Trichlorethylen, Chlorbenzol oder Chlortoluol, oder andere Lösungsmittel wie Acetonitril, Essigsäure, *N,N*-Dimethylformamid (DMF), Dimethylsulfoxid (DMSO), *N,N*'-Dimethylpropylenharnstoff (DMPU) oder *N*-Methylpyrrolidon (NMP). Ebenso ist es möglich, Gemische der genannten Lösungsmittel zu verwenden. Bevorzugt wird Ethanol eingesetzt.

Als Säuren sind für den Verfahrensschritt (VI) → (VII) insbesondere organische Säuren wie Essigsäure, Trifluoressigsäure, Methansulfonsäure, Trifluormethansulfonsäure oder p-Toluolsulfonsäure geeignet; bevorzugt wird Essigsäure verwendet.

Die Umsetzung (VI) → (VII) erfolgt im Allgemeinen in einem Temperaturbereich von +20°C bis +150°C, bevorzugt bei +60°C bis +120°C. Die Reaktion kann bei normalem, erhöhtem oder bei vermindertem Druck durchgeführt werden (z.B. von 0.5 bis 5 bar). Im Allgemeinen arbeitet man bei Normaldruck.

Inerte Lösungsmittel für die Verfahrensschritte (VII) + (VIII) → (I-A1), (VII) + (IX) → (I-A1) und (VII) + (X) → (I-A2) sind beispielsweise Ether wie Diethylether, Methyl-*tert*.-butylether, Dioxan, Tetrahydrofuran, Glykoldimethylether oder Diethylenglykoldimethylether, Kohlenwasserstoffe wie Benzol, Toluol, Xylol, Hexan, Cyclohexan oder Erdölfraktionen, Halogenkohlenwasserstoffe wie Dichlormethan, Trichlormethan, Tetrachlormethan, 1,2-Dichlorethan, Trichlorethan, Tetrachlorethan, Trichlorethylen, Chlorbenzol oder Chlortoluol, oder andere Lösungsmittel wie *N,N-*Dimethylformamid (DMF), Dimethylsulfoxid (DMSO), *N,N'*-Dimethylpropylenharnstoff (DMPU), *N*-Methylpyrrolidon (NMP), Pyridin oder Acetonitril. Ebenso ist es möglich, Gemische der genannten Lösungsmittel zu verwenden. Bevorzugt werden im Verfahrensschritt (VII) + (VIII) → (I-A1) Tetrahydrofuran oder Dimethylformamid, im Verfahrensschritt (VII) + (IX) → (I-A1) Dichlormethan und im Verfahrensschritt (VII) + (X) → (I-A2) Pyridin eingesetzt.

Als Basen für den Verfahrensschritt (VII) + (VIII) → (I-A1) eignen sich insbesondere Alkali- oder Erdalkalicarbonate wie Lithium-, Natrium-, Kalium-, Calcium- oder Cäsiumcarbonat, Alkalihydride wie Natrium- oder Kaliumhydrid, Amide wie Lithium-, Natrium- oder Kalium-bis-(trimethylsilyl)amid oder Lithiumdiisopropylamid, metallorganische Verbindungen wie Butyllithium oder Phenyllithium, oder auch Phosphazen-Basen wie beispielsweise P2-t-Bu oder P4-t-Bu [so genannte "Schwesinger-Basen", vgl. R. Schwesinger, H. Schlemper, Angew. Chem. Int. Ed. Engl. 26, 1167 (1987); T. Pietzonka, D. Seebach, Chem. Ber. 124, 1837 (1991)]. Bevorzugt wird Natriumhydrid oder die Phosphazen-Base P4-t-Bu verwendet.

Als Basen für den Verfahrensschritt (VII) + (X) → (I-A2) eignen sich insbesondere Alkali- oder Erdalkalicarbonate wie Lithium-, Natrium-, Kalium-, Calcium- oder Cäsiumcarbonat, Alkalihydride wie Natrium- oder Kaliumhydrid, metallorganische Verbindungen wie Butyllithium oder Phenyllithium, oder organische Amine wie Triethylamin, *N*-Methylmorpholin, N-Methylpiperidin, *N,N*-Diisopropylethylamin, Pyridin, 1,5-Diazabicyclo[4.3.0]non-5-en (DBN), 1,8-Diazabicyclo-[5.4.0]undec-7-en (DBU) oder 1,4-Diazabicyclo[2.2.2]octan (DABCO^{®}). Bevorzugt wird Pyridin verwendet, das gleichzeitig auch als Lösungsmittel dient.

Der Verfahrensschritt (VII) + (IX) → (I-A1) wird im Allgemeinen ohne Zusatz einer Base durchgeführt.

Die Umsetzungen (VII) + (VIII) → (I-A1), (VII) + (IX) → (I-A1) und (VII) + (X) → (I-A2) erfolgen im Allgemeinen in einem Temperaturbereich von -20°C bis +100°C, bevorzugt bei 0°C bis +50°C. Die Reaktionen können bei normalem, erhöhtem oder bei vermindertem Druck durchgeführt werden (z.B. von 0.5 bis 5 bar). Im Allgemeinen arbeitet man bei Normaldruck.

Die Verbindungen der Formel (II) sind konmlerziell erhältlich, literaturbekannt oder können in Analogie zu literaturbekannten Verfahren hergestellt werden (vgl. nachfolgende Reaktionsschemata 1-7). Die Verbindungen der Formeln (III), (IV), (VIII), (IX) und (X) sind vielfach kommerziell erhältlich, literaturbekannt oder nach literaturbekannten Methoden herstellbar.

Weiter beschrieben ist ein Verfahren zur Herstellung der erfindungsgemäßen Verbindungen der Formel (I-B) 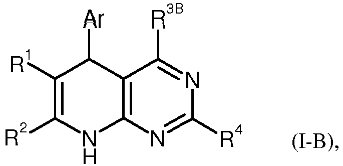 in welcher Ar, R¹, R² und R⁴ jeweils die oben angegebenen Bedeutungen haben
und
- R^{3B}: für (C₁-C₆)-Alkoxy oder (C₁-C₆)-Alkylthio, welche jeweils mit (C₃-C₇)-Cycloalkyl substituiert sein können, oder für Trifluormethoxy, Amino, Mono-(C₁-C₆)-alkylamino oder eine
Gruppe der Formel -O-SO₂-R¹⁶ steht, worin R¹⁶ die oben angegebene Bedeutung hat,
dadurch gekennzeichnet, dass man eine Verbindung der Formel (II) 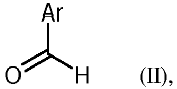 in welcher Ar die oben angegebene Bedeutung hat,
mit einer Verbindung der Formel (XI) 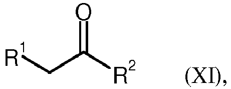 in welcher R¹ und R² die oben angegebenen Bedeutungen haben,
zu einer Verbindung der Formel (XII) 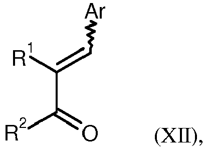 in welcher Ar, R¹ und R² jeweils die oben angegebenen Bedeutungen haben,
kondensiert und diese anschließend entweder

### [B-1] in einem inerten Lösungsmittel mit einer Verbindung der Formel (XIII)

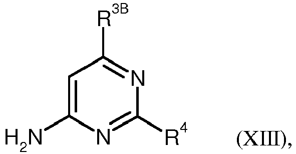

in welcher R^{3B} und R⁴ die oben angegebenen Bedeutungen haben,
umsetzt
oder

### [B-2] zunächst in einem inerten Lösungsmittel mit einer Verbindung der Formel (XIV)

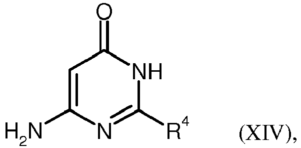

in welcher R⁴ die oben angegebene Bedeutung hat,
zu einer Verbindung der Formel (XV) 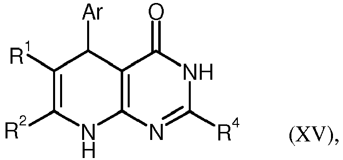 in welcher Ar, R¹, R² und R⁴ jeweils die oben angegebenen Bedeutungen haben,
umsetzt und diese dann in einem inerten Lösungsmittel gegebenenfalls in Gegenwart einer Base mit einer Verbindung der Formel (VIII) oder einem Trialkyloxonium-Salz der Formel (IX)

R¹⁷-Q (VIII)

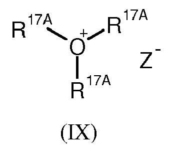

in welchen
- R¹⁷: für (C₁-C₆)-Alkyl, das mit (C₃-C₇)-Cycloalkyl substituiert sein kann, oder für Trifluormethyl steht,
- R^{17A}: für Methyl oder Ethyl steht,
- Q: für eine Abgangsgruppe, wie beispielsweise Halogen, Mesylat, Tosylat oder Triflat, steht
und
- Z⁻: für ein nicht-nukleophiles Anion, wie beispielsweise Tetrafluoroborat, steht,
zu Verbindungen der Formel (I-B1) 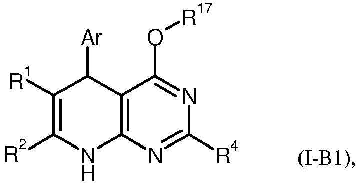
in welcher Ar, R¹, R², R⁴ und R¹⁷ jeweils die oben angegebenen Bedeutungen haben, alkyliert
oder die Verbindungen der Formel (XV) in einem inerten Lösungsmittel in Gegenwart einer Base mit einer Verbindung der Formel (X) 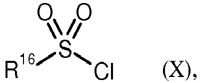 in welcher R¹⁶ die oben angegebene Bedeutung hat,
zu Verbindungen der Formel (I-B2) 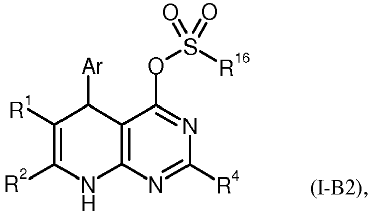 in welcher Ar, R¹, R², R⁴ und R¹⁶ jeweils die oben angegebenen Bedeutungen haben, umsetzt
und gegebenenfalls die jeweils resultierenden Verbindungen der Formel (I-B), (I-B1) bzw. (I-B2) nach dem Fachmann bekannten Methoden in ihre Enantiomere und/oder Diastereomere trennt und/oder mit den entsprechenden (i) Lösungsmitlen und/oder (ii) Basen oder Säuren in ihre Solvate, Salze und/oder Solvate der Salze überführt.

Der Verfahrensschritt (II) + (XI) → (XII) erfolgt im Allgemeinen in einem inerten Lösungsmittel, gegebenenfalls in Gegenwart einer Säure und/oder Base, in einem Temperaturbereich von +20°C bis zum Siedepunkt des Lösungsmittels bei Normaldruck.

Als inerte Lösungsmittel eignen sich hierbei beispielsweise Halogenkohlenwasserstoffe wie Dichlormethan, Trichlormethan, Tetrachlormethan, Trichlorethan oder 1,2-Dichlorethan, oder andere Lösungsmittel wie Acetonitril, Eisessig, Pyridin, Benzol, Chlorbenzol, Toluol oder Xylol. Bevorzugt erfolgt die Umsetzung in Dichlormethan oder Toluol bei der jeweiligen Rückfluss-Temperatur unter Normaldruck.

Die Reaktion (II) + (XI) → (XII) wird vorteilhafterweise in Gegenwart einer Säure in Kombination mit Piperidin oder Pyridin als Base und/oder einem wasserentziehenden Mittel, wie beispielsweise Molekularsieb, durchgeführt. Als Säuren eignen sich beispielsweise Essigsäure oder p-Toluolsulfonsäure. Bevorzugt ist eine Reaktionsführung unter Zusatz von Piperidiniumacetat.

Inerte Lösungsmittel für die Verfahrensschritte (XII) + (XIII) → (I-B) bzw. (XII) + (XIV) → (XV) sind beispielsweise Alkohole wie Methanol, Ethanol, n-Propanol, Isopropanol, n-Butanol oder *tert*.-Butanol, oder andere Lösungsmittel wie Acetonitril, Tetrahydrofuran, Dioxan, 1,2-Dimethoxyethan, Toluol oder Eisessig. Die Umsetzungen erfolgen im Allgemeinen in einem Temperaturbereich von +50°C bis +120°C. Bevorzugt werden die Reaktionen in Ethanol oder Isopropanol bei der jeweiligen Rückfluss-Temperatur unter Normaldruck durchgeführt.

Inerte Lösungsmittel für die Verfahrensschritte (XV) + (VIII) → (I-B1), (XV) + (IX) → (I-B1) und (XV) + (X) → (I-B2) sind beispielsweise Ether wie Diethylether, Methyl-*tert*.-butylether, Dioxan, Tetrahydrofuran, Glykoldimethylether oder Diethylenglykoldimethylether, Kohlenwasserstoffe wie Benzol, Toluol, Xylol, Hexan, Cyclohexan oder Erdölfraktionen, Halogenkohlenwasserstoffe wie Dichlormethan, Trichlormethan, Tetrachlormethan, 1,2-Dichlorethan, Trichlorethan, Tetrachlorethan, Trichlorethylen, Chlorbenzol oder Chlortoluol, oder andere Lösungsmittel wie *N,N-*Dimethylformamid (DMF), Dimethylsulfoxid (DMSO), *N,N'*-Dimethylpropylenharnstoff (DMPU), *N*-Methylpyrrolidon (NMP), Pyridin oder Acetonitril. Ebenso ist es möglich, Gemische der genannten Lösungsmittel zu verwenden. Bevorzugt werden im Verfahrensschritt (XV) + (VIII) → (I-B1) Tetrahydrofuran oder Dimethylformamid, im Verfahrensschritt (XV) + (IX) → (I-B1) Dichlormethan und im Verfahrensschritt (XV) + (X) → (I-B2) Pyridin eingesetzt.

Als Basen für den Verfahrensschritt (XV) + (VIII) → (I-B1) eignen sich insbesondere Alkali- oder Erdalkalicarbonate wie Lithium-, Natrium-, Kalium-, Calcium- oder Cäsiumcarbonat, Alkalihydride wie Natrium- oder Kaliumhydrid, Amide wie Lithium-, Natrium- oder Kalium-bis-(trimethylsilyl)amid oder Lithiumdiisopropylamid, metallorganische Verbindungen wie Butyllithium oder Phenyllithium, oder auch Phosphazen-Basen wie beispielsweise P2-t-Bu oder P4-t-Bu [so genannte "Schwesinger-Basen", vgl. R. Schwesinger, H. Schlemper, Angew. Chem. Int. Ed. Eng/. 26, 1167 (1987); T. Pietzonka, D. Seebach, Chem. Ber. 124, 1837 (1991)]. Bevorzugt wird Natriumhydrid oder die Phosphazen-Base P4-t-Bu verwendet.

Als Basen für den Verfahrensschritt (XV) + (X) → (I-B2) eignen sich insbesondere Alkali- oder Erdalkalicarbonate wie Lithium-, Natrium-, Kalium-, Calcium- oder Cäsiumcarbonat, Alkalihydride wie Natrium- oder Kaliumhydrid, metallorganische Verbindungen wie Butyllithium oder Phenyllithium, oder organische Amine wie Triethylamin, *N*-Methylmorpholin, N-Methylpiperidin, *N,N*-Diisopropylethylamin, Pyridin, 1,5-Diazabicyclo[4.3.0]non-5-en (DBN), 1,8-Diazabicyclo-[5.4.0]undec-7-en (DBU) oder 1,4-Diazabicyclo[2.2.2]octan (DABCO^{®}). Bevorzugt wird Pyridin verwendet, das gleichzeitig auch als Lösungsmittel dient.

Der Verfahrensschritt (XV) + (IX) → (I-B1) wird im Allgemeinen ohne Zusatz einer Base durchgeführt.

Die Umsetzungen (XV) + (VIII) → (I-B1), (XV) + (IX) → (I-B1) und (XV) + (X) → (I-B2) erfolgen im Allgemeinen in einem Temperaturbereich von -20°C bis +100°C, bevorzugt bei 0°C bis +50°C. Die Reaktionen können bei normalem, erhöhtem oder bei vermindertem Druck durchgeführt werden (z.B. von 0.5 bis 5 bar). Im Allgemeinen arbeitet man bei Normaldruck.

Die Verbindungen der Formel (II) sind konmlerziell erhältlich, literaturbekannt oder können in Analogie zu literaturbekannten Verfahren hergestellt werden (vgl. nachfolgende Reaktionsschemata 1-7). Die Verbindungen der Formeln (XIII) und (XIV) sind zum Teil konmlerziell erhältlich oder aber literaturbekannt oder können nach Literaturverfahren hergestellt werden (vgl. Reaktionsschema 9 und dort zitierte Literatur).

Die Verbindungen der Formeln (VIII), (IX), (X) und (XI) sind vielfach kommerziell erhältlich, literaturbekannt oder nach literaturbekannten Methoden herstellbar.

Die Herstellung der erfindungsgemäßen Verbindungen kann durch die folgenden Syntheseschemata veranschaulicht werden: 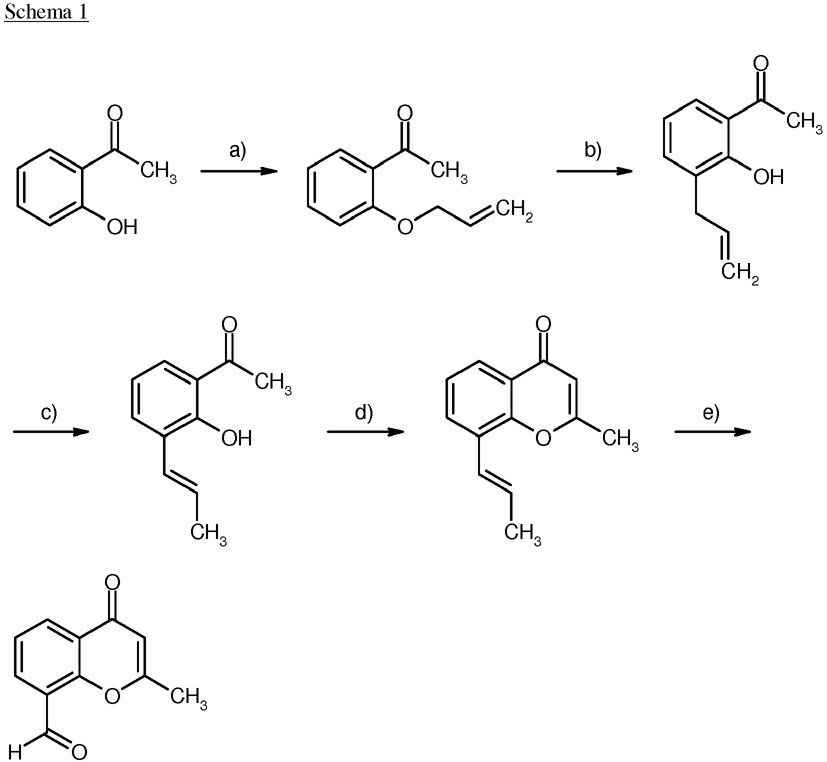
[a): Allylbromid, Kaliumcarbonat, cat. Kaliumiodid, Aceton, Rückfluss; b): 230°C, 4 h; c): Bis-(benzonitril)dichlorpalladium(II), Toluol, 120°C, 16 h; d): Acetylchlorid, Natriumhydrid, THF, 10-25°C, 16 h; e): 1. Ozon, Dichlormethan, -60°C, 30 min; 2. Dimethylsulfid]. 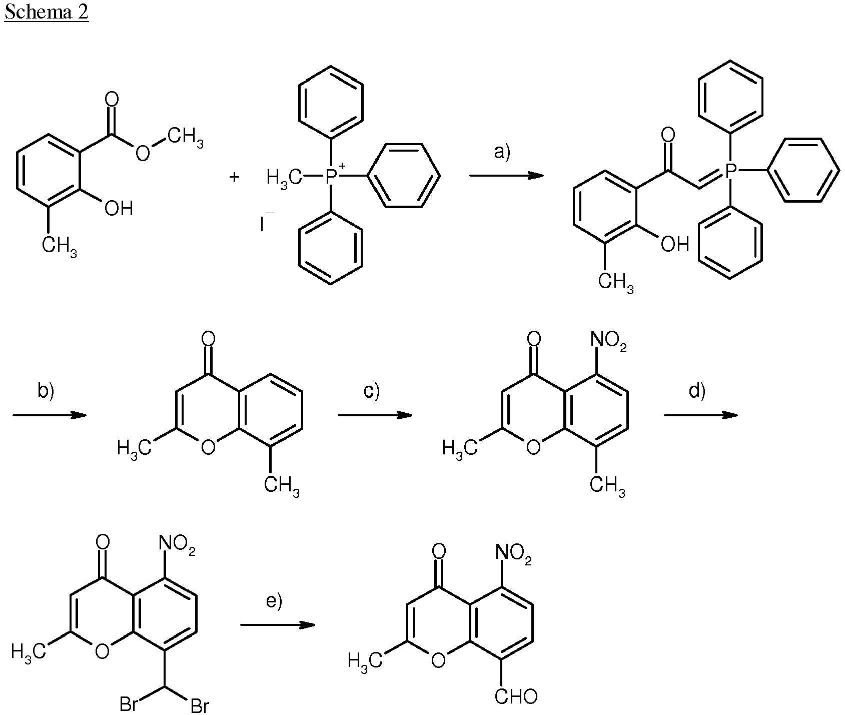
[a): n-Butyllithium, THF, 60°C, 3 h; b): Essigsäureanhydrid, Pyridin, Rückfluss, 6 h; c): konz. H,SO₄, HNO₃, 0°C, 1 h; d): *N*-Bromsuccinimid, AIBN, Tetrachlorkohlenstoff, Rückfluss; e): *N-*Methylmorpholin-N-oxid, Acetonitril, Rückfluss]. 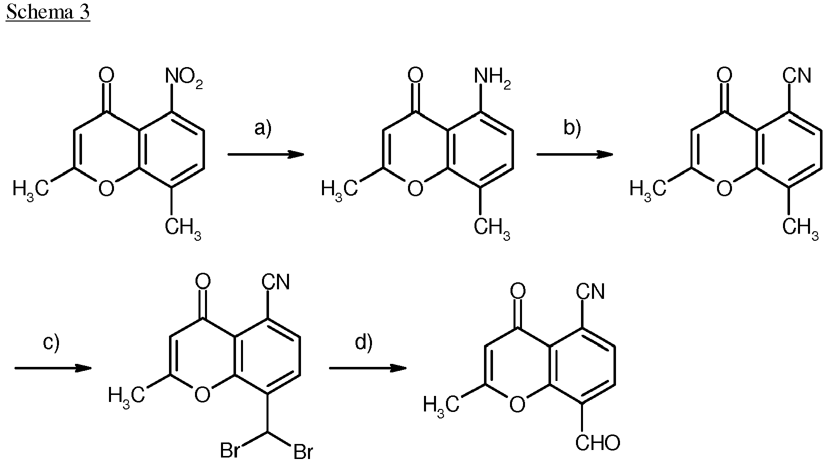
[a): Zinn(II)chlorid-Dihydrat, Ethylacetat, 70°C; b): 1. Natriumnitrit, Schwefelsäure, 0°C, 1.5 h; 2. Kupfer(I)cyanid, Natriumcyanid, Wasser/Ethylacetat, 0°C, 45 min; c): *N*-Bromsuccinimid, AIBN, Tetrachlorkohlenstoff, Rückfluss; d): *N*-Methylmorpholin-*N*-oxid, Acetonitril, Rückfluss]. 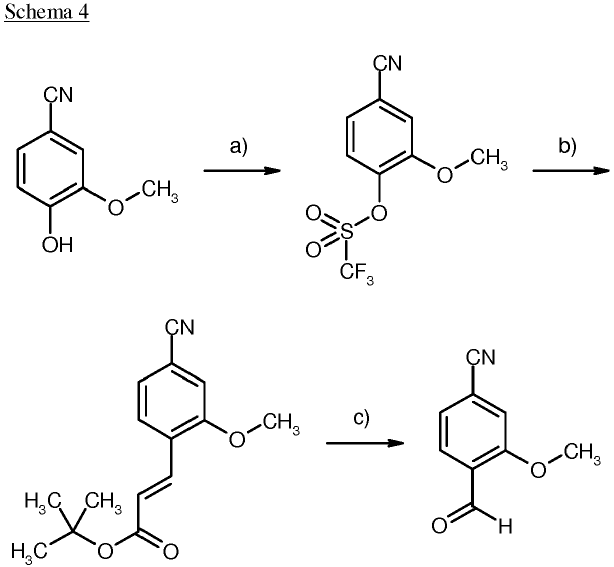
[a): Trifluormethansulfonsäureanhydrid, Pyridin, 0°C → RT, 30 min; b): Acrylsäure-*tert*.-butylester, Bis(triphenylphosphin)dichlorpalladium(II), DMF, 120°C, 24 h; c): cat. Osmiumtetroxid, cat. Benzyltriethylammoniumchlorid, Natriumperiodat, THF/Wasser, 20-25°C, 2 h]. 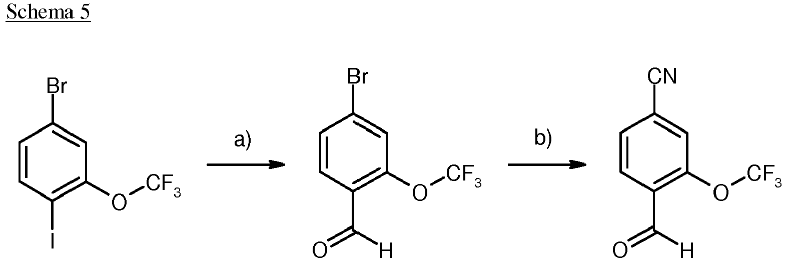
[a): n-Butyllithium, THF, -78°C, dann *N*-Formylmorpholin; b): Zinkcyanid, Tetrakis(triphenyl-phosphin)palladium(0), DMF, Mikrowelle 250°C / 5 min]. 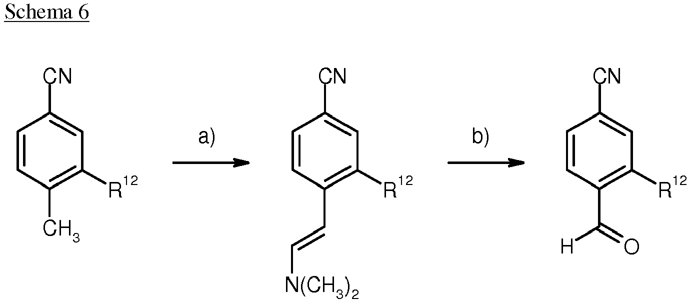
[a): *N,N*-Dimethylformamid-dimethylacetal, DMF, 140-180°C; b): Natriumperiodat, THF/Wasser]. 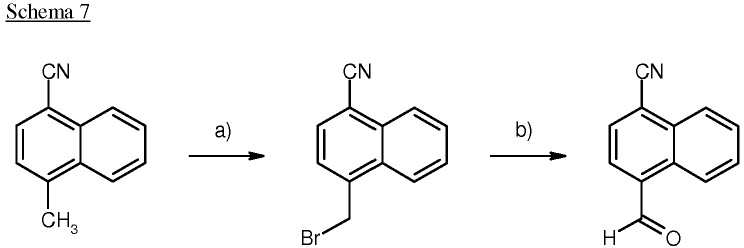
[a): *N*-Bromsuccinimid, 2,2'-Azobis-2-methylpropannitril, Tetrachlormethan, Rückfluss; b): *N-*Methylmorpholin-N-oxid, Acetonitril, 3Å-Molekularsieb]. 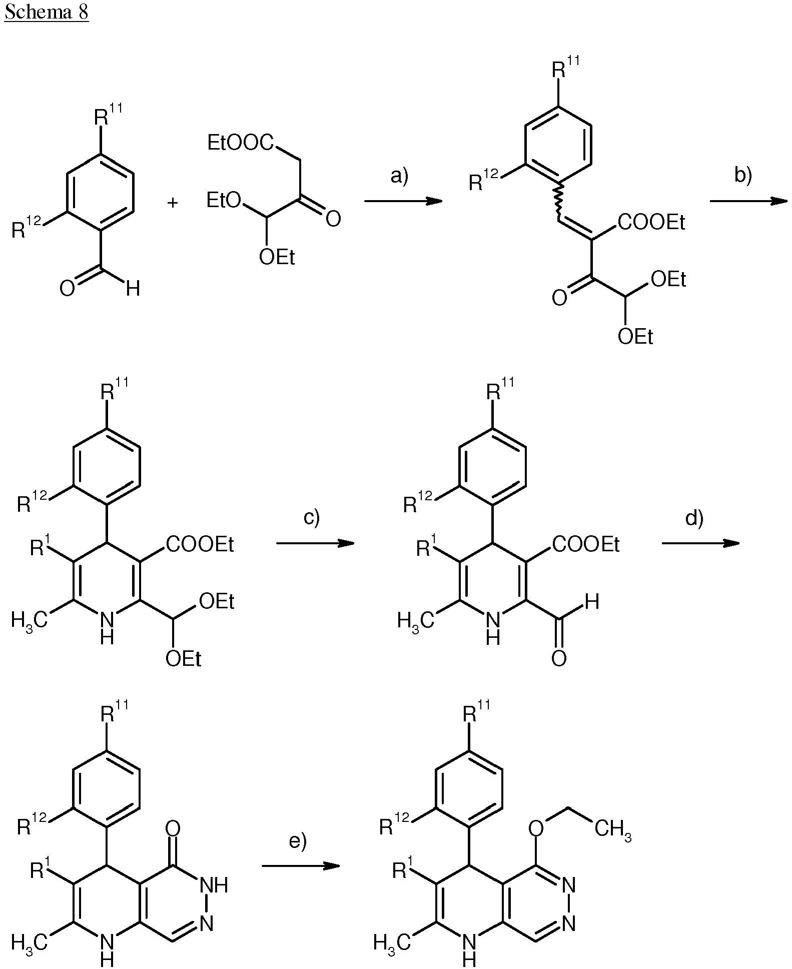
[a): Essigsäure, Piperidin, Dichlormethan, Rückfluss; b): 4-Amino-3-penten-2-on (R¹ = CH₃-CO-) oder Ethyl 3-aminobut-2-enoat (R¹ = EtOOC-), Isopropanol, Rückfluss; c): Salzsäure, Aceton, RT; d): Hydrazin-Hydrat, Ethanol/Essigsäure, 100°C; e): Triethyloxoniumtetrafluoroborat, Dichlormethan, RT]. 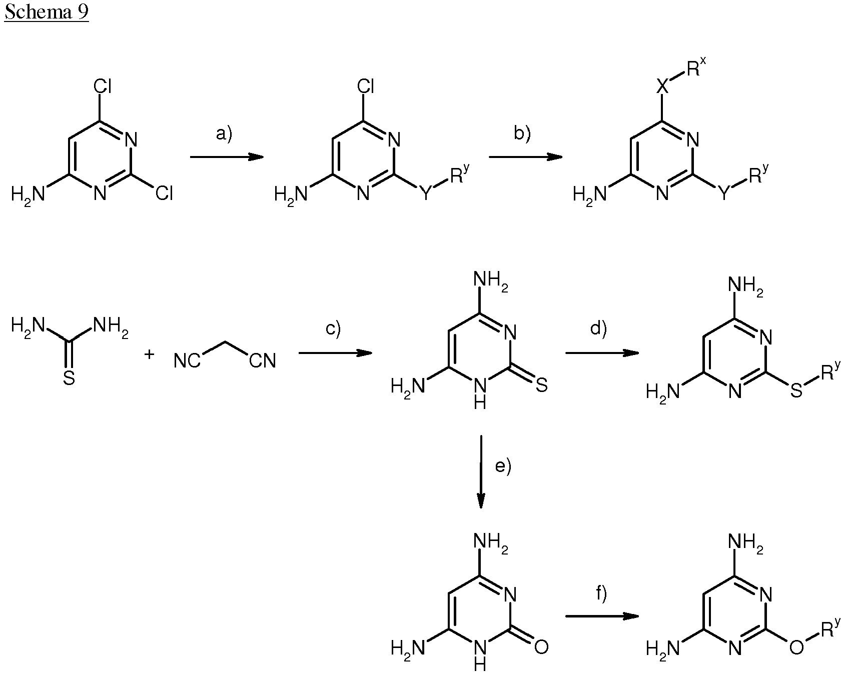 [X, Y = N, O oder S; a): R^{y}-YH, Base; vgl. z.B. R.A. Nugent et al., J. Med. Chem. 1998, 41, 3793-3803. b): R^{x}-XH, Base; vgl. z.B. P. Manesiotis et al., J. Org. Chem. 2005, 70, 2729-2738 (X = N); B. Roth et al., J. Am. Chem. Soc. 1951, 73, 2864-2868 (X = O). c): NaOEt, EtOH; siehe A. Bendich et al., J. Am. Chem. Soc. 1948, 70, 3109-3113. d): R^{y}-I, EtOH oder R^{y}-I, K₂CO₃, Aceton; vgl. z.B. E.C. Taylor, C.K. Cain, J. Am. Chem. Soc. 1952, 74, 1644-1647. e): Chloressigsäure, Schwefelsäure; vgl. z.B. A. Bendich et al., J. Am. Chem. Soc. 1948, 70, 3109-3113. f): (R^{y})₃O⁺ BF₄⁻, Dichlormethan]. 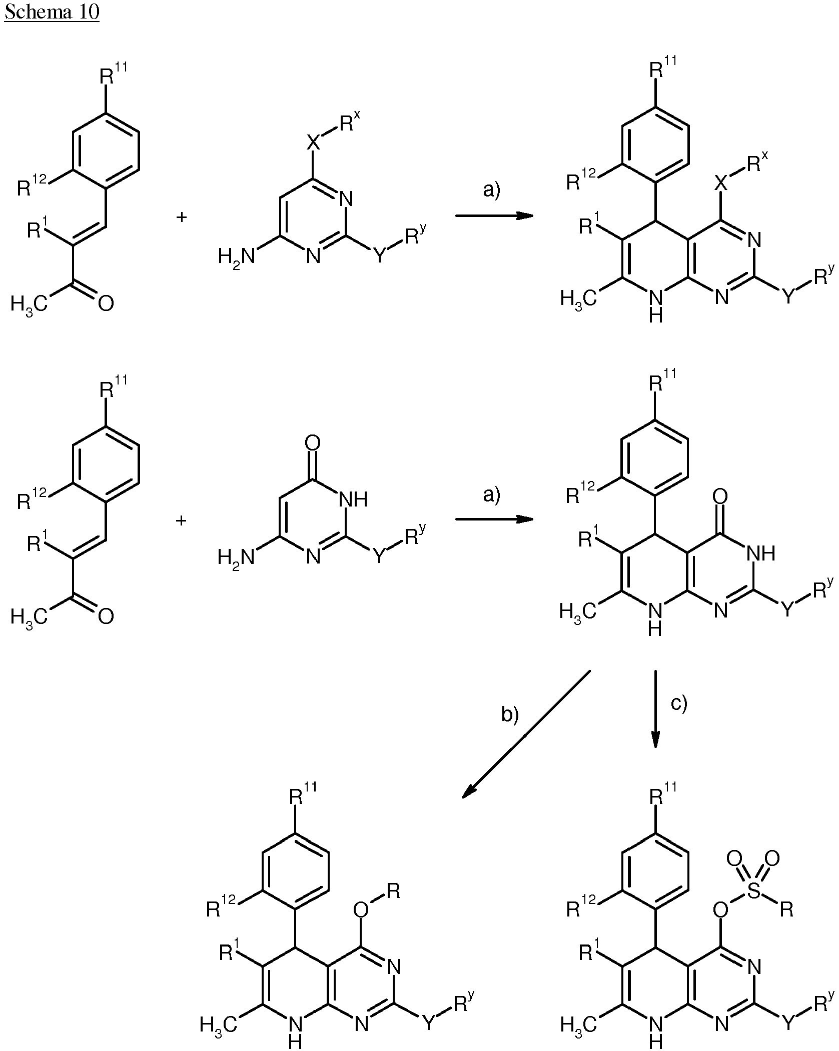 [X, Y = N, O oder S; a): Isopropanol, Rückfluss, 12 h; b): R₃O⁺ BF₄⁻, Dichlormethan, RT, 2-12 h; c): R-SO₂-Cl, Pyridin, RT, 1-3 h].

Die erfindungsgemäßen Verbindungen wirken als Antagonisten des Mineralokorticoid-Rezeptors und zeigen ein nicht vorhersehbares, wertvolles pharmakologisches Wirkspektrum. Sie eignen sich daher zur Verwendung als Arzneimittel zur Behandlung und/oder Prophylaxe von Krankheiten bei Menschen und Tieren.

Die erfindungsgemäßen Verbindungen sind geeignet für die Prophylaxe und/oder Behandlung von verschiedenen Erkrankungen und krankheitsbedingten Zuständen, insbesondere von Erkrankungen, die entweder durch eine Erhöhung der Aldosteron-Konzentration im Plasma oder durch eine Veränderung der Aldosteron-Plasmakonzentration relativ zur Renin-Plasmakonzentration gekennzeichnet sind oder mit diesen Veränderungen einhergehen. Beispielsweise seien genannt: idiopathischer primärer Hyperaldosteronismus, Hyperaldosteronismus bei Nebennierenhyperplasie, Nebennierenadenomen und/oder Nebennierencarzinomen, Hyperaldosteronismus bei Leberzirrhose, Hyperaldosteronismus bei Herzinsuffizienz sowie (relativer) Hyperaldosteronismus bei essentieller Hypertonie.

Die erfindungsgemäßen Verbindungen sind aufgrund ihres Wirkmechanismus ferner geeignet für die Prophylaxe des plötzlichen Herztodes bei Patienten, die unter einem erhöhten Risiko stehen, an einem plötzlichen Herztod zu versterben. Dies sind insbesondere Patienten, die z.B. an einer der folgenden Erkrankungen leiden: Hypertonie, Herzinsuffizienz, koronare Herzerkrankung, stabile und instabile Angina pectoris, myokardiale Ischämie, Myokardinfarkt, dilatative Kardiomyopathien, Schock, Arteriosklerose, atriale und ventrikuläre Arrhythmie, transitorische und ischämische Attacken, Hirnschlag, entzündliche kardiovaskuläre Erkrankungen, periphere und kardiale Gefäßerkrankungen, periphere Durchblutungsstörungen, pulmonale Hypertonie, Spasmen der Koronararterien und peripherer Arterien, Thrombosen, thromboembolische Erkrankungen sowie Vaskulitis.

Die erfindungsgemäßen Verbindungen können ferner verwendet werden für die Prophylaxe und/ oder Behandlung von Ödembildung wie zum Beispiel pulmonales Ödem, renales Ödem oder Herzinsuffizienz-bedingtes Ödem, und von Restenosen wie nach Thrombolysetherapien, percutantransluminalen Angioplastien (PTA) und transluminalen Koronarangioplastien (PTCA), Herztransplantationen und Bypass-Operationen.

Weiterhin eignen sich die erfindungsgemäßen Verbindungen zur Verwendung als Diuretikum und bei Elektrolytstörungen wie zum Beispiel Hyperkalzämie.

Außerdem können die erfindungsgemäßen Verbindungen eingesetzt werden für die Prophylaxe und/oder Behandlung von Diabetes mellitus und diabetischen Folgeerkrankungen wie z.B. Neuropathie und Nephropathie, von akutem und chronischem Nierenversagen sowie der chronischen Niereninsuffizienz.

Weiterer Gegenstand der vorliegenden Erfindung ist die Verwendung der erfindungsgemäßen Verbindungen zur Herstellung eines Arzneimittels zur Behandlung und/oder Prävention von Erkrankungen, insbesondere der zuvor genannten Erkrankungen.

Weiterer Gegenstand der vorliegenden Erfindung ist eine Verbindung wie in Anspruch 1 definiert zur Behandlung und/oder Prävention von Erkrankungen, insbesondere der zuvor genannten Erkrankungen, unter Verwendung einer wirksamen Menge von mindestens einer der erfindungsgemäßen Verbindungen. Die erfindungsgemäßen Verbindungen können allein oder bei Bedarf in Kombination mit anderen Wirkstoffen eingesetzt werden. Weiterer Gegenstand der vorliegenden Erfindung sind Arzneimittel, enthaltend mindestens eine der erfindungsgemäßen Verbindungen und einen oder mehrere weitere Wirkstoffe, insbesondere zur Behandlung und/oder Prävention der zuvor genannten Erkrankungen. Als geeignete Kombinationswirkstoffe seien beispielhaft und vorzugsweise genannt:
- den Blutdruck senkende Wirkstoffe, beispielhaft und vorzugsweise aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker und Rho-Kinase-Inhibitoren;
- Diuretika, insbesondere Schleifendiuretika sowie Thiazide und Thiazid-ähnliche Diuretika;
- antithrombotisch wirkende Mittel, beispielhaft und vorzugsweise aus der Gruppe der Thrombozytenaggregationshemmer, der Antikoagulantien oder der profibrinolytischen Substanzen;
- den Fettstoffwechsel verändernde Wirkstoffe, beispielhaft und vorzugsweise aus der Gruppe der Thyroidrezeptor-Agonisten, Cholesterinsynthese-Inhibitoren wie beispielhaft und vorzugsweise HMG-CoA-Reduktase- oder Squalensynthese-Inhibitoren, der ACAT-Inhibitoren, CETP-Inhibitoren, MTP-Inhibitoren, PPAR-alpha-, PPAR-gamma- und/oder PPAR-delta-Agonisten, Cholesterin-Absorptionshemmer, Lipase-Inhibitoren, polymeren Gallensäureadsorber, Gallensäure-Reabsorptionshemmer und Lipoprotein(a)-Antagonisten;
- organische Nitrate und NO-Donatoren, wie beispielsweise Natriumnitroprussid, Nitroglycerin, Isosorbidmononitrat, Isosorbiddinitrat, Molsidomin oder SIN-1, sowie inhalatives NO;
- positiv-inotrop wirksame Verbindungen, wie beispielsweise Herzglycoside (Digoxin), betaadrenerge und dopaminerge Agonisten wie Isoproterenol, Adrenalin, Noradrenalin, Dopamin und Dobutamin;
- Verbindungen, die den Abbau von cyclischem Guanosinmonophosphat (cGMP) und/oder cyclischem Adenosinmonophosphat (cAMP) inhibieren, wie beispielsweise Inhibitoren der Phosphodiesterasen (PDE) 1, 2, 3, 4 und/oder 5, insbesondere PDE 5-Inhibitoren wie Sildenafil, Vardenafil und Tadalafil, sowie PDE 3-Inhibitoren wie Amrinone und Milrinone;
- natriuretische Peptide, wie z.B. "atrial natriuretic peptide" (ANP, Anaritide), "B-type natriuretic peptide" oder "brain natriuretic peptide" (BNP, Nesiritide), "C-type natriuretic peptide" (CNP) sowie Urodilatin;
- Calcium-Sensitizer, wie beispielhaft und vorzugsweise Levosimendan;
- Kalium-Supplements;
- NO-unabhängige, jedoch Häm-abhängige Stimulatoren der Guanylatcyclase, wie insbesondere die in WO 00/06568, WO 00/06569, WO 02/42301 und WO 03/095451 beschriebenen Verbindungen;
- NO- und Häm-unabhängige Aktivatoren der Guanylatcyclase, wie insbesondere die in WO 01/19355, WO 01/19776, WO 01/19778, WO 01/19780, WO 02/070462 und WO 02/070510 beschriebenen Verbindungen;
- Inhibitoren der humanen neutrophilen Elastase (HNE), wie beispielsweise Sivelestat oder DX-890 (Reltran);
- die Signaltransduktionskaskade inhibierende Verbindungen, wie beispielsweise Tyrosinkinase-Inhibitoren, insbesondere Sorafenib, Imatinib, Gefitinib und Erlotinib; und/oder
- den Energiestoffwechsel des Herzens beeinflussende Verbindungen, wie beispielhaft und vorzugsweise Etomoxir, Dichloracetat, Ranolazine oder Trimetazidine.

Bei einer bevorzugten Ausführungsform der Erfindung werden die erfindungsgemäßen Verbindungen in Kombination mit einem Diuretikum, wie beispielhaft und vorzugsweise Furosemid, Bumetanid, Torsemid, Bendroflumethiazid, Chlorthiazid, Hydrochlorthiazid, Hydroflumethiazid, Methyclothiazid, Polythiazid, Trichlormethiazid, Chlorthalidon, Indapamid, Metolazon, Quinethazon, Acetazolamid, Dichlorphenamid, Methazolamid, Glycerin, Isosorbid, Mannitol, Amilorid oder Triamteren, verabreicht.

Unter den Blutdruck senkenden Mitteln werden vorzugsweise Verbindungen aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Rho-Kinase-Inhibitoren sowie der Diuretika verstanden.

Bei einer bevorzugten Ausführungsform der Erfindung werden die erfindungsgemäßen Verbindungen in Kombination mit einem Calcium-Antagonisten, wie beispielhaft und vorzugsweise Nifedipin, Amlodipin, Verapamil oder Diltiazem, verabreicht.

Bei einer bevorzugten Ausführungsform der Erfindung werden die erfindungsgemäßen Verbindungen in Kombination mit einem Angiotensin AII-Antagonisten, wie beispielhaft und vorzugsweise Losartan, Candesartan, Valsartan, Telmisartan oder Embusartan, verabreicht.

Bei einer bevorzugten Ausführungsform der Erfindung werden die erfindungsgemäßen Verbindungen in Kombination mit einem ACE-Hemmer, wie beispielhaft und vorzugsweise Enalapril, Captopril, Lisinopril, Ramipril, Delapril, Fosinopril, Quinopril, Perindopril oder Trandopril, verabreicht.

Bei einer bevorzugten Ausführungsform der Erfindung werden die erfindungsgemäßen Verbindungen in Kombination mit einem Endothelin-Antagonisten, wie beispielhaft und vorzugsweise Bosentan, Darusentan, Ambrisentan oder Sitaxsentan, verabreicht.

Bei einer bevorzugten Ausführungsform der Erfindung werden die erfindungsgemäßen Verbindungen in Kombination mit einem Renin-Inhibitor, wie beispielhaft und vorzugsweise Aliskiren, SPP-600 oder SPP-800, verabreicht.

Bei einer bevorzugten Ausführungsform der Erfindung werden die erfindungsgemäßen Verbindungen in Kombination mit einem alpha-1-Rezeptoren-Blocker, wie beispielhaft und vorzugsweise Prazosin, verabreicht.

Bei einer bevorzugten Ausführungsform der Erfindung werden die erfindungsgemäßen Verbindungen in Kombination mit einem beta-Rezeptoren-Blocker, wie beispielhaft und vorzugsweise Propranolol, Atenolol, Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, verabreicht.

Bei einer bevorzugten Ausführungsform der Erfindung werden die erfindungsgemäßen Verbindungen in Kombination mit einem Rho-Kinase-Inhibitor, wie beispielhaft und vorzugsweise Fasudil, Y-27632, SLx-2119, BF-66851, BF-66852, BF-66853, KI-23095 oder BA-1049, verabreicht.

Unter antithrombotisch wirkenden Mitteln (Antithrombotika) werden vorzugsweise Verbindungen aus der Gruppe der Thrombozytenaggregationshemmer, der Antikoagulantien oder der profibrinolytischen Substanzen verstanden.

Bei einer bevorzugten Ausführungsform der Erfindung werden die erfindungsgemäßen Verbindungen in Kombination mit einem Thrombozytenaggregationshemmer, wie beispielhaft und vorzugsweise Aspirin, Clopidogrel, Ticlopidin oder Dipyridamol, verabreicht.

Bei einer bevorzugten Ausführungsform der Erfindung werden die erfindungsgemäßen Verbindungen in Kombination mit einem Thrombin-Inhibitor, wie beispielhaft und vorzugsweise Ximelagatran, Melagatran, Bivalirudin oder Clexane, verabreicht.

Bei einer bevorzugten Ausführungsform der Erfindung werden die erfindungsgemäßen Verbindungen in Kombination mit einem GPIIb/IIIa-Antagonisten, wie beispielhaft und vorzugsweise Tirofiban oder Abciximab, verabreicht.

Bei einer bevorzugten Ausführungsform der Erfindung werden die erfindungsgemäßen Verbindungen in Kombination mit einem Faktor Xa-Inhibitor, wie beispielhaft und vorzugsweise Rivaroxaban (BAY 59-7939), DU-176b, Apixaban, Otamixaban, Fidexaban, Razaxaban, Fondaparinux, Idraparinux, PMD-3112, YM-150, KFA-1982, EMD-503982, MCM-17, MLN-1021, DX 9065a, DPC 906, JTV 803, SSR-126512 oder SSR-128428, verabreicht.

Bei einer bevorzugten Ausführungsform der Erfindung werden die erfindungsgemäßen Verbindungen in Kombination mit Heparin oder einem low molecular weight (LMW)-Heparin-Derivat verabreicht.

Bei einer bevorzugten Ausführungsform der Erfindung werden die erfindungsgemäßen Verbindungen in Kombination mit einem Vitamin K-Antagonisten, wie beispielhaft und vorzugsweise Coumarin, verabreicht.

Unter den Fettstoffwechsel verändernden Mitteln werden vorzugsweise Verbindungen aus der Gruppe der CETP-Inhibitoren, Thyroidrezeptor-Agonisten, Cholesterinsynthese-Inhibitoren wie HMG-CoA-Reduktase- oder Squalensynthese-Inhibitoren, der ACAT-Inhibitoren, MTP-Inhibitoren, PPAR-alpha-, PPAR-gamma- und/oder PPAR-delta-Agonisten, Cholesterin-Absorptionshemmer, polymeren Gallensäureadsorber, Gallensäure-Reabsorptionshemmer, Lipase-Inhibitoren sowie der Lipoprotein(a)-Antagonisten verstanden.

Bei einer bevorzugten Ausführungsform der Erfindung werden die erfindungsgemäßen Verbindungen in Kombination mit einem CETP-Inhibitor, wie beispielhaft und vorzugsweise Torcetrapib (CP-529 414), JJT-705, BAY 60-5521, BAY 78-7499 oder CETP-vaccine (Avant), verabreicht.

Bei einer bevorzugten Ausführungsform der Erfindung werden die erfindungsgemäßen Verbindungen in Kombination mit einem Thyroidrezeptor-Agonisten, wie beispielhaft und vorzugsweise D-Thyroxin, 3,5,3'-Trüodothyronin (T3), CGS 23425 oder Axitirome (CGS 26214), verabreicht.

Bei einer bevorzugten Ausführungsform der Erfindung werden die erfindungsgemäßen Verbindungen in Kombination mit einem HMG-CoA-Reduktase-Inhibitor aus der Klasse der Statine, wie beispielhaft und vorzugsweise Lovastatin, Simvastatin, Pravastatin, Fluvastatin, Atorvastatin, Rosuvastatin, Cerivastatin oder Pitavastatin, verabreicht.

Bei einer bevorzugten Ausführungsform der Erfindung werden die erfindungsgemäßen Verbindungen in Kombination mit einem Squalensynthese-Inhibitor, wie beispielhaft und vorzugsweise BMS-188494 oder TAK-475, verabreicht.

Bei einer bevorzugten Ausführungsform der Erfindung werden die erfindungsgemäßen Verbindungen in Kombination mit einem ACAT-Inhibitor, wie beispielhaft und vorzugsweise Avasimibe, Melinamide, Pactimibe, Eflucimibe oder SMP-797, verabreicht.

Bei einer bevorzugten Ausführungsform der Erfindung werden die erfindungsgemäßen Verbindungen in Kombination mit einem MTP-Inhibitor, wie beispielhaft und vorzugsweise Implitapide, BMS-201038, R-103757 oder JTT-130, verabreicht.

Bei einer bevorzugten Ausführungsform der Erfindung werden die erfindungsgemäßen Verbindungen in Kombination mit einem PPAR-gamma-Agonisten, wie beispielhaft und vorzugsweise Pioglitazone oder Rosiglitazone, verabreicht.

Bei einer bevorzugten Ausführungsform der Erfindung werden die erfindungsgemäßen Verbindungen in Kombination mit einem PPAR-delta-Agonisten, wie beispielhaft und vorzugsweise GW-501516 oder BAY 68-5042, verabreicht.

Bei einer bevorzugten Ausführungsform der Erfindung werden die erfindungsgemäßen Verbindungen in Kombination mit einem Cholesterin-Absorptionshemmer, wie beispielhaft und vorzugsweise Ezetimibe, Tiqueside oder Pamaqueside, verabreicht.

Bei einer bevorzugten Ausführungsform der Erfindung werden die erfindungsgemäßen Verbindungen in Kombination mit einem Lipase-Inhibitor, wie beispielhaft und vorzugsweise Orlistat, verabreicht.

Bei einer bevorzugten Ausführungsform der Erfindung werden die erfindungsgemäßen Verbindungen in Kombination mit einem polymeren Gallensäureadsorber, wie beispielhaft und vorzugsweise Cholestyramin, Colestipol, Colesolvam, CholestaGel oder Colestimid, verabreicht.

Bei einer bevorzugten Ausführungsform der Erfindung werden die erfindungsgemäßen Verbindungen in Kombination mit einem Gallensäure-Reabsorptionshemmer, wie beispielhaft und vorzugsweise ASBT (= IBAT)-Inhibitoren wie z.B. AZD-7806, S-8921, AK-105, BARI-1741, SC-435 oder SC-635, verabreicht.

Bei einer bevorzugten Ausführungsform der Erfindung werden die erfindungsgemäßen Verbindungen in Kombination mit einem Lipoprotein(a)-Antagonisten, wie beispielhaft und vorzugsweise Gemcabene calcium (CI-1027) oder Nicotinsäure, verabreicht.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukonmlen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen und Akronyme:

- abs.: absolut
- cat.: katalytisch
- CI: chemische Ionisation (bei MS)
- d: Tag(e)
- DC: Dünnschichtchromatographie
- DMF: Dimethylformamid
- DMSO: Dimethylsulfoxid
- d. Th.: der Theorie (bei Ausbeute)
- EI: Elektronenstoß-Ionisation (bei MS)
- ent: Enantiomer / enantiomerenrein
- eq: Äquivalent(e)
- ESI: Elektrospray-Ionisation (bei MS)
- Et: Ethyl
- GC-MS: Gaschromatographie-gekoppelte Massenspektrometrie
- h: Stunde(n)
- HPLC: Hochdruck-, Hochleistungsflüssigchromatographie
- konz.: konzentriert
- LC-MS: Flüssigchromatographie-gekoppelte Massenspektrometrie
- min: Minute(n)
- MS: Massenspektrometrie
- NMR: Kernresonanzspektrometrie
- R_{f}: Retentionsindex (bei DC)
- Rₜ: Retentionszeit (bei HPLC)
- RT: Raumtemperatur
- THF: Tetrahydrofuran
- v/v: Volumen-zu-Volumen-Verhältnis (einer Lösung)

### LC-MS-, GC-MS- und HPLC-Methoden:

### Methode 1 (LC-MS):

Gerätetyp MS: Micromass ZQ; Gerätetyp HPLC: Waters Alliance 2795; Säule: Phenomenex Synergi 2µ Hydro-RP Mercury 20 mm x 4 nm; Eluent A: 11 Wasser + 0.5 ml 50%-ige Ameisensäure, Eluent B: 11 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

### Methode 2 (LC-MS):

Gerätetyp MS: Micromass ZQ; Gerätetyp HPLC: HP 1100 Series; UV DAD; Säule: Phenomenex Synergi 2µ Hydro-RP Mercury 20 mm x 4 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 man/3.0 min/4.5 min 2 ml/min; Ofen: 50°C; UV-Detektion: 210 nm.

### Methode 3 (LC-MS):

Instrument: Micromass Quattro LCZ mit HPLC Agilent Serie 1100; Säule: Phenomenex Synergi 2µ Hydro-RP Mercury 20 mm x 4 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 208-400 nm.

### Methode 4 (LC-MS):

Gerätetyp MS: Micromass ZQ; Gerätetyp HPLC: HP 1100 Series; UV DAD; Säule: Phenomenex Gemini 3µ 30 mm x 3.00 mm; Eluent A: 11 Wasser + 0.5 ml 50%-ige Ameisensäure, Eluent B: 1 1 Acetonitril + 0.5 ml 50%-ige Ameisensäure; Gradient: 0.0 min 90% A → 2.5 min 30% A → 3.0 min 5% Au 4.5 min 5% A; Fluss: 0.0 min 1 ml/min → 2.5 min/3.0 min/4.5 min 2 ml/min; Ofen: 50°C; UV-Detektion: 210 nm.

### Methode 5 (LC-MS):

Instrument: Micromass Platform LCZ mit HPLC Agilent Serie 1100; Säule: Thermo Hypersil GOLD 3µ 20 mm x 4 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 100% A → 0.2 min 100% A → 2.9 min 30% A → 3.1 min 10% A → 5.5 min 10% A; Ofen: 50°C; Fluss: 0.8 ml/min; UV-Detektion: 210 nm.

### Methode 6 (LC-MS):

Instrument: Micromass Platform LCZ mit HPLC Agilent Serie 1100; Säule: Thermo HyPURITY Aquastar 3µ 50 mm x 2.1 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 100% A → 0.2 min 100% A → 2.9 min 30% A → 3.1 min 10% A → 5.5 min 10% A; Ofen: 50°C; Fluss: 0.8 ml/min; UV-Detektion: 210 nm.

### Methode 7 (GC-MS):

Instrument: Micromass GCT, GC 6890; Säule: Restek RTX-35MS, 30 m x 250 µm x 0.25 µm; konstanter Fluss mit Helium: 0.88 ml/min; Ofen: 60°C; Inlet: 250°C; Gradient: 60°C (0.30 min halten), 50°C/min → 120°C, 16°C/min → 250°C, 30°C/min → 300°C (1.7 min halten).

### Methode 8 (HPLC):

Instrument: HP 1100 mit DAD-Detektion; Säule: Kromasil 100 RP-18, 60 mm x 2.1 mm, 3.5 µm; Eluent A: 5 ml HClO₄ (70%-ig) / Liter Wasser, Eluent B: Acetonitril; Gradient: 0 min 2% B → 0.5 min 2% B → 4.5 min 90% B → 9 min 90% B → 9.2 min 2% B → 10 min 2% B; Fluss: 0.75 ml/min; Säulentemperatur: 30°C; UV-Detektion: 210 nm.

### Methode 9 (chirale HPLC):

Säule: 250 mm x 46 mm, basierend auf dem chiralen Selektor Poly(*N*-methacryloyl-D-leucin-*tert*.-butylamin); Eluent: Isohexan/Ethylacetat 1:1; Temperatur: 24°C; Fluss: 2 ml/min; UV-Detektion: 260 nm.

### Methode 10 (chirale HPLC):

Säule: Daicel Chiralpak AD-H, 5 µm, 250 mm x 4 mm; Eluent: Isohexan/Isopropanol 80:20; Temperatur: 35°C; Fluss: 2 ml/min; UV-Detektion: 250 nm.

### Methode 11 (LC-MS):

Instrument: Micromass Quattro LCZ mit HPLC Agilent Serie 1100; Säule: Phenomenex Gemini 3µ 30 mm x 3.00 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 208-400 nm.

### Ausgangsverbindungen und Intermediate:

### Beispiel 1A

### 1-[2-(Allyloxy)phenyl]ethanon

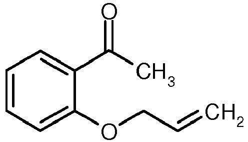

542 g (3.9 mol) 2-Hydroxyacetophenon werden mit 592 g (4.9 mol) Allylbromid, 1000 g (7.2 mol) Kaliumcarbonat und 13.2 g (79 mmol) Kaliumiodid in 2.4 Liter Aceton 24 h lang zum Rückfluss erhitzt. Nach Abkühlen auf Raumtemperatur wird filtriert und das Lösungsmittel im Vakuum entfernt. Der Rückstand wird in Toluol gelöst und mit 10%-iger Natronlauge und Wasser gewaschen. Nach Einengen werden 689 g (98% d. Th.) der Titelverbindung erhalten.
¹H-NMR (300 MHz, CDCl₃): δ = 2.68 (s, 3H), 4.68 (dd, 2H), 5.89 (dd, 2H), 6.09 (m, 1H), 6.99 (dd, 2H), 7.44 (m, 1H), 7.71 (d, 1H).

### Beispiel 2A

### 1-(3-Allyl-2-hydroxyphenyl)ethanon

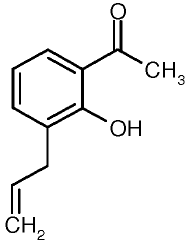

160 g (0.9 mol) 1-[2-(Allyloxy)phenyl]ethanon werden im Metallbad 4 h lang bei 230-240°C gerührt. Nach Abkühlen auf Raumtemperatur wird das Produkt über einen Dünnschichtverdampfer bei 140°C und 0.4 mbar destilliert. Es werden 155 g (97% d. Th.) der Titelverbindung erhalten.
¹H-NMR (300 MHz, CDCl₃): δ = 2.68 (s, 3H), 3.44 (d, 2H), 5.09 (m, 2H), 6.01 (m, 1H), 6.85 (t, 1H), 7.38 (dd, 1H), 7.62 (dd, 1H), 12.61 (s, 1H).

### Beispiel 3A

### 1-{2-Hydroxy-3-[(1E)-prop-1-en-1-yl]phenyl}ethanon

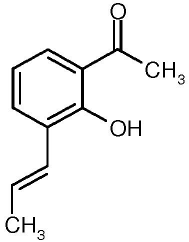

40 g (227 mmol) 1-(3-Allyl-2-hydroxyphenyl)ethanon werden in 120 ml Toluol gelöst und mit 2.17 g (5.6 mmol) Bis(benzonitril)dichlorpalladium(II) versetzt. Die Reaktionsmischung wird über Nacht auf 120°C erhitzt. Nach Abkühlen auf Raumtemperatur wird über Kieselgur filtriert und das Lösungsmittel im Vakuum entfernt. Es werden 20.9 g (95% d.Th.) der Titelverbindung erhalten, welche ohne weitere Reinigung in der nächsten Stufe umgesetzt wird.
LC-MS (Methode 1): Rₜ = 2.36 min; [M+H]⁺ = 177
¹H-NMR (300 MHz, CDCl₃): δ = 1.91 (dd, 3H), 2.63 (s, 3H), 6.32 (m, 1H), 6.73 (dd, 1H), 6.85 (t, 1H), 7.59 (m, 2H), 12.74 (s, 1H).

### Beispiel 4A

### 2-Methyl-8-[(1E)-prop-1-en-1-yl]-4H-chromen-4-on

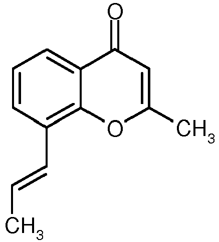

12.52 g (313.2 mmol) 60%-iges Natriumhydrid (Suspension in Mineralöl) werden unter Argon bei 10°C in 300 ml absolutem THF vorgelegt. Zu der Suspension werden 18.4 g (104.4 mmol) 1-{2-Hydroxy-3-[(1*E*)-prop-1-en-1-yl]phenyl}ethanon langsam zugetropft. Nach 15 min werden 9 g (114.9 mmol) Acetylchlorid zugegeben. Die Reaktionsmischung wird über Nacht bei Raumtemperatur gerührt. Man hydrolysiert mit 300 ml Wasser und extrahiert mehrfach mit Ethylacetat. Nach Waschen der organischen Phase mit gesättigter Natriumchlorid-Lösung wird über Natriumsulfat getrocknet. Anschließend wird das Lösungsmittel im Vakuum entfernt. Der Rückstand wird in 200 ml Methanol aufgenommen und mit 50 ml 20%-iger Salzsäure 30 min auf 80°C erhitzt. Anschließend wird das Lösungsmittel im Vakuum entfernt und der Rückstand mit 400 ml Wasser versetzt. Es wird mehrfach mit Dichlormethan extrahiert. Nach Trocknen der organischen Phase über Magnesiumsulfat wird das Lösungsmittel im Vakuum entfernt und der Rückstand mittels Säulenchromatographie gereinigt (Laufmittel: Dichlormethan/Methanol 98:2). Es werden 10.5 g (50.2% d. Th.) der Titelverbindung als gelbes Öl erhalten.
LC-MS (Methode 3): Rₜ = 2.07 min; [M+H]⁺ = 201
¹H-NMR (300 MHz, CDCl₃): δ = 1.98 (dd, 3H), 2.43 (s, 3H), 6.18 (s, 1H), 6.40 (m, 1H), 6.85 (dd, 1H), 7.31 (t, 1H), 7.72 (dd, 1H), 8.05 (dd, 1H).

### Beispiel 5A

### 2-Methyl-4-oxo-4H-chromen-8-carbaldehyd

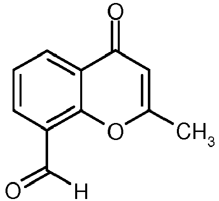

18.5 g (62.8 mmol) 2-Methyl-8-[(1*E*)-prop-1-en-1-yl]-4*H*-chromen-4-on werden in 400 ml Dichlormethan gelöst und auf -60°C abgefühlt. In die Reaktionslösung wird 30 min lang Ozon eingeleitet. Anschließend wird die Reaktionsmischung mit Dimethylsulfid versetzt. Nach Erwärmen auf Raumtemperatur wird das Lösungsmittel im Vakuum entfernt und der Rückstand mit wenig Methanol aufgeschlämmt. Nach Filtration wird der verbleibende Feststoff aus Diethylether umkristallisiert. Es werden 9.1 g (77.4% d. Th.) der Titelverbindung erhalten.
LC-MS (Methode 1): Rₜ = 1.31 min; [M+H]⁺= 189
¹H-NMR (300 MHz, CDCl₃): δ = 2.48 (s, 3H), 6.27 (s, 1H), 7.51 (m, 1H), 8.21 (dd, 1H), 8.46 (dd, 1H), 10.67 (s, 1H).

### Beispiel 6A

### 3-[(2-Methyl-4-oxo-4H-chromen-8-yl)methylen]pentan-2,4-dion

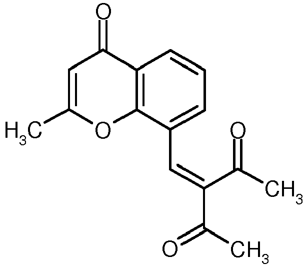

20 g (106 mmol) 2-Methyl-4-oxo-4*H*-chromen-8-carbaldehyd, 12 ml (116 mmol) 2,4-Pentandion, 9.1 ml (159 mmol) Essigsäure und 0.21 ml (2.1 mmol) Piperidin in 400 ml wasserfreiem Dichlormethan werden 24 h lang am Wasserabscheider unter Rückfluss gerührt. Nach dem Abkühlen wird die Reaktionslösung nacheinander mit gesättigter Natriumhydrogencarbonat-Lösung und gesättigter Natriumchlorid-Lösung gewaschen. Die organische Phase wird über Magnesiumsulfat getrocknet und eingeengt. Der Rückstand wird aus Isopropanol umkristallisiert. Man erhält 24.3 g (73% d. Th.) der Titelverbindung als weißen Feststoff.
LC-MS (Methode 4): Rₜ = 1.91 min; [M+H]⁺ = 271
¹H-NMR (300 MHz, DMSO-d₆): δ = 2.24 (s, 3H), 2.44 (s, 3H), 2.54 (s, 3H), 6.33 (s, 1H), 7.49 (t, 1H), 7.64 (dd, 1H), 7.97 (s, 1H), 8.07 (dd, 1H).

### Beispiel 7A

### Ethyl 2-[(2-methyl-4-oxo-4H-chromen-8-yl)methylen]-3-oxobutanoat

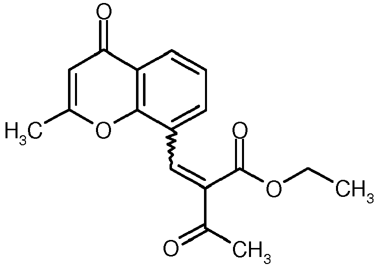

5 g (26.57 mmol) 2-Methyl-4-oxo-4*H*-chromen-8-carbaldehyd, 3.4 ml (26.57 mmol) Ethyl 3-oxo-butanoat, 1.9 ml (33.21 mmol) Essigsäure und 263 µl (2.66 mmol) Piperidin in 50 ml wasserfreiem Dichlormethan werden 24 h lang am Wasserabscheider unter Rückfluss gerührt. Nach dem Abkühlen wird die Lösung mit Dichlormethan (50 ml) verdünnt und nacheinander mit gesättigter Natriumhydrogencarbonat-Lösung und gesättigter Natriumchlorid-Lösung gewaschen. Die organische Phase wird über Magnesiumsulfat getrocknet und eingeengt. Der Rückstand wird aus Isopropanol umkristallisiert. Man erhält 7.63 g (91% d. Th.) der Titelverbindung als E/Z-Gemisch.
LC-MS (Methode 3): Rₜ = 1.91 und 2.03 min; [M+H]⁺ = 301
¹H-NMR (300 MHz, DMSO-d₆): δ = 1.04 (t, 1.5H), 1.28 (t, 1.5H), 2.34 (s, 1.5H), 2.42 (s, 1.5H), 2.49 (s, 1.5H), 2.55 (s, 1.5H), 4.14 (q, 1H), 4.29 (q, 1H), 6.32 (s, 0.5H), 6.33 (s, 0.5H), 7.47 (t, 0.5H), 7.52 (t, 0.5H), 7.65 (dd, 0.5H), 7.65 (dd, 0.5H), 7.98 (s, 0.5H), 8.07 (dd, 0.5H), 8.08 (s, 0.5H), 8.09 (dd, 0.5H).

### Beispiel 8A

### 4-Brom-2-(trifluormethoxy)benzaldehyd

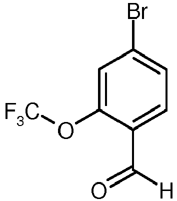

20.00 g (54.51 mmol) 4-Brom-2-(trifluormethoxy)iodbenzol werden in 200 ml THF gelöst und auf -78°C gekühlt. Anschließend werden 26.16 ml (65.41 mmol) einer 2.5 M Lösung von n-Butyllithium in Hexan zugetropft. Es wird 30 min nachgerührt und anschließend 14.43 g (125.37 mmol) *N*-Formylmorpholin zudosiert. Nachdem vollständiger Umsatz detektiert ist (DC-Kontrolle), wird bei -78°C mit Isopropanol solvolysiert. Nach dem Erwärmen auf Raumtemperatur wird mit Wasser versetzt und zweimal mit Dichlormethan extrahiert. Die vereinigten organischen Phasen werden mit gesättigter Natriumchlorid-Lösung gewaschen, mit Natriumsulfat getrocknet und das Lösungsmittel unter vermindertem Druck abdestilliert. Der Rückstand wird säulenchromatographisch gereinigt (Kieselgel, Laufmittel: Cyclohexan/Essigsäureethylester 5:1). Es werden 11.43 g (78% d. Th.) der Titelverbindung erhalten.
GC-MS (Methode 7): Rt = 4.24 min; MS (EIpos): m/z = 270 [M+H]⁺
¹H-NMR (300 MHz, DMSO-d₆): δ = 7.85-7.92 (m, 3H), 10.20 (s, 1H).

### Beispiel 9A

### 4-Formyl-3-(trifluormethoxy)benzonitril

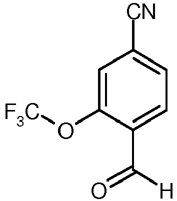

10.63 g (39.51 mmol) 4-Brom-2-(trifluormethoxy)benzaldehyd, 3.43 g (29.24 mmol) Zinkcyanid und 1.37 g (1.19 mmol) Tetrakis(triphenylphosphin)palladium(0) werden in 80 ml DMF gelöst. Anschließend wird die Reaktionsmischung in mehreren Portionen in einer Single Mode-Mikrowelle (Emrys Optimizer, 5 min bei 220°C) umgesetzt. Die vereinigten Ansätze werden mit Wasser versetzt und zweimal mit Toluol extrahiert. Die vereinigten organischen Phasen werden mit gesättigter Natriumchlorid-Lösung gewaschen, mit Natriumsulfat getrocknet und anschließend das Lösungsmittel am Rotationsverdampfer entfernt. Der Rückstand wird säulenchromatographisch aufgereinigt (Kieselgel, Laufmittel: Cyclohexan/Essigsäureethylester 10:1). Es werden 3.32 g (78% d. Th.) der Titelverbindung in 80%-iger Reinheit (nach LC-MS) erhalten.
MS (EIpos): m/z = 215 [M]⁺
¹H-NMR (300 MHz, DMSO-d₆): δ = 7.85-7.91 (m, 3H), 10.20 (s, 1H).

### Beispiel 10A

### Natrium 1-cyanoprop-1-en-2-olat

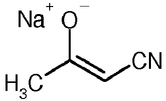

Natrium (7.69 g, 335 mmol) wird portionsweise in 350 ml wasserfreies Methanol eingetragen. Nach dem Abkühlen der Reaktionsmischung auf 25°C wird 5-Methylisoxazol (27.8 g, 335 mmol) langsam und portionsweise zugegeben (exotherme Reaktion). Nach beendeter Zugabe wird der Ansatz für 4 h bei RT gerührt und anschließend eingeengt. Der Rückstand wird mit wenig Diethylether gewaschen, abgesaugt und im Ölpumpenvakuum getrocknet. Man erhält 32.0 g (91% d. Th.) der Titelverbindung.
¹H-NMR (400 MHz, DMSO-d₆): δ = 3.18 (s, 1H), 1.51 (s, 3H).

### Beispiel 11A

### 4-Cyano-2-methoxyphenyl-trifluormethansulfonat

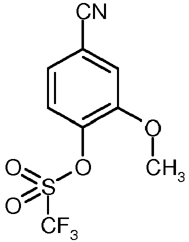

Zu einer Lösung von 20 g (134 mmol) 4-Hydroxy-3-methoxybenzonitril in Pyridin (80 ml) werden langsam 24 ml (141 mmol) Trifluormethansulfonsäureanhydrid getropft, wobei die Reaktionstemperatur mit Hilfe eines Eisbads unter 25°C gehalten wird. Die Suspension wird dann 1 h bei RT gerührt. Eiswasser (400 ml) wird zugegeben und die Suspension noch bis zum Erreichen der Raumtemperatur weiter gerührt. Dann wird filtriert, der Feststoff in Ethylacetat gelöst und diese Lösung mit gesättigter Natriumchlorid-Lösung gewaschen. Die organische Phase wird über Magnesiumsulfat getrocknet und eingeengt. Es werden 37.13 g (92% d. Th.) der Titelverbindung als weißer Feststoff erhalten.
LC-MS (Methode 4): Rₜ = 2.54 min; MS (EIpos): m/z = 282 [M+H]⁺
¹H-NMR (300 MHz, DMSO-d₆): δ = 3.97 (s, 3H), 7.60 (dd, 1H), 7.71 (d, 1H), 7.92 (d, 1H).

### Beispiel 12A

### tert.-Butyl (2E)-3-(4-cyano-2-methoxyphenyl)acrylat

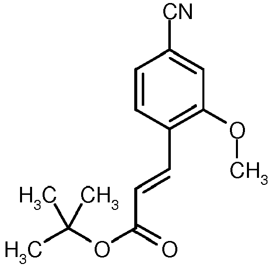

Zu einer entgasten Lösung von 37.13 g (132 mmol) 4-Cyano-2-methoxyphenyl-trifluormethansulfonat, 35 ml (245 mmol) *tert*-Butylacrylat und 90 ml (645 mmol) Triethylamin in DMF (250 ml) werden 4 g (5.7 mmol) Bis(triphenylphosphin)palladium(II)chlorid hinzugefügt. Die Lösung wird unter Schutzgasatmosphäre 24 h lang bei 100°C gerührt. Anschließend wird Eiswasser (1000 ml) zugegeben und die Suspension mit Ethylacetat (3 x 100 ml) extrahiert. Die organische Phase wird mit gesättigter Natriumchlorid-Lösung gewaschen, über Magnesiumsulfat getrocknet und eingeengt. Der Rückstand wird säulenchromatographisch gereinigt (Kieselgel, Laufmittel: Cyclohexan-Essigsäureethylester 10:1). Es werden 24.6 g (72% d. Th.) der Titelverbindung als weißer Feststoff erhalten.
LC-MS (Methode 1): Rₜ = 2.59 min; MS (EIpos): m/z = 260 [M+H]⁺
¹H-NMR (300 MHz, DMSO-d₆): δ = 1.48 (s, 9H), 3.93 (s, 3H), 6.65 (d, 1H), 7.42 (d, 1H), 7.58 (s, 1H), 7.74 (d, 1H), 7.89 (d, 1H).

### Beispiel 13A

### 4-Formyl-3-methoxybenzonitril

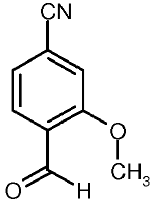

Zu einer kräftig gerührten Lösung von 48 g (185 mmol) *tert*.-Butyl (2*E*)-3-(4-cyano-2-methoxyphenyl)acrylat, 207 mg (0.81 mmol) Osmiumtetroxid und 1.4 g (6.14 mmol) Benzyltriethylammoniumchlorid in 750 ml Wasser/THF (2:1) werden 79 g (370 mmol) Natriummetaperiodat portionsweise zugegeben, wobei die Reaktionstemperatur unter 30°C gehalten wird. Die Lösung wird 1 h bei RT weitergerührt. Wasser (2000 ml) wird zugegeben und die Mischung anschließend filtriert. Der verbleibende Feststoff wird in Ethylacetat gelöst und die Lösung mit gesättigter Natriumchlorid-Lösung gewaschen. Die organische Phase wird über Magnesiumsulfat getrocknet und eingeengt. Der Rückstand wird mit Petrolether verrührt. Es werden 21.18 g (71% d. Th.) der Titelverbindung als weißer Feststoff erhalten.
LC-MS (Methode 4): Rₜ = 1.87 min; MS (EIpos): m/z = 162 [M+H]⁺
¹H-NMR (300 MHz, DMSO-d₆): δ = 3.98 (s, 3H), 7.53 (d, 1H), 7.80 (s, 1H), 7.81 (d, 1H), 10.37 (s, 1H).

### Beispiel 14A

### 4-(2-Acetyl-3-oxobut-1-en-1-yl)-3-methoxybenzonitril

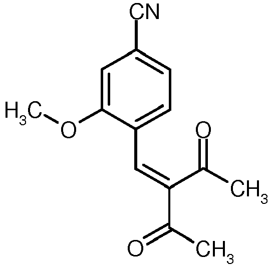

21 g (130 mmol) 4-Formyl-3-methoxybenzonitril, 14.7 ml (143 mmol) 2,4-Pentandion, 11.2 ml (195 mmol) Essigsäure und 2.6 ml (26 mmol) Piperidin in 400 ml trockenem Dichlormethan werden 24 h lang am Wasserabscheider unter Rückfluss gerührt. Nach dem Abkühlen wird die Reaktionslösung nacheinander mit gesättigter Natriumhydrogencarbonat-Lösung und gesättigter Natriumchlorid-Lösung gewaschen. Die organische Phase wird über Magnesiumsulfat getrocknet und eingeengt. Der Rückstand wird aus Diethylether umkristallisiert. Man erhält 23.2 g (92% d. Th.) der Titelverbindung als leicht braunen Feststoff.
LC-MS (Methode 4): Rₜ = 2.05 min; [M+H]⁺ = 244
¹H-NMR (300 MHz, DMSO-d₆): δ = 2.20 (s, 3H), 2.42 (s, 3H), 3.89 (s, 3H), 7.37 (d, 1H), 7.46 (dd, 1H), 7.60 (d, 1H), 7.68 (s, 1H).

### Beispiel 15A

### 4-(2-Acetyl-3-oxobut-1-en-1-yl)-benzonitril

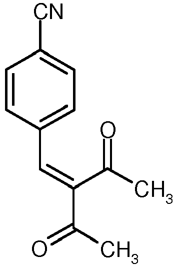

2.3 g (17.5 mmol) 4-Formylbenzonitril, 1.98 ml (19.29 mmol) 2,4-Pentandion, 1 ml (26 mmol) Essigsäure und 0.34 ml (3.5 mmol) Piperidin in 40 ml wasserfreiem Dichlormethan werden 24 h lang am Wasserabscheider unter Rückfluss gerührt. Nach dem Abkühlen wird die Reaktionslösung nacheinander mit gesättigter Natriumhydrogencarbonat-Lösung und gesättigter Natriumchlorid-Lösung gewaschen. Die organische Phase wird über Magnesiumsulfat getrocknet und eingeengt. Der Rückstand wird aus Diethylether umkristallisiert. Man erhält 3.18 g (85% d. Th.) der Titelverbindung als leicht braunen Feststoff.
¹H-NMR (300 MHz, DMSO-d₆): δ = 2.26 (s, 3H), 2.46 (s, 3H), 7.60 (d, 2H), 7.76 (s, 1H), 7.93 (d, 2H).

### Beispiel 16A

### 9-Oxo-9H-fluoren-4-carbaldehyd

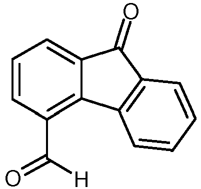

Unter Argon wird 9-Oxo-9*H*-fluoren-4-carbonsäuremethylester (9.85 g, 41.3 mmol) in 180 ml wasserfreiem THF vorgelegt. Bei RT wird innerhalb von 90 min RED-AL® (38 ml, 136 mmol) [Natrium-bis-(2-methoxyethoxy)aluminiumdihydrid, 70%-ige Lösung in Toluol] zugetropft und das Reaktionsgemisch 1 h nachgerührt. Der Ansatz wird durch vorsichtige, tropfenweise Zugabe von 15 ml Wasser hydrolysiert. Anschließend gibt man 60 ml 6 N Salzsäure zu und extrahiert mit Essigsäureethylester (4 x je 150 ml). Die vereinigten organischen Phasen werden mit gesättigter Natriumchlorid-Lösung gewaschen (2 x je 100 ml), über Natriumsulfat getrocknet und am Rotationsverdampfer eingeengt. Man erhält 12.1 g des korrespondierenden Alkohols. Von diesem werden 8.77 g (41.3 mmol) in 200 ml Dioxan gelöst und mit aktiviertem Mangandioxid (25.1 g, 289 mmol) versetzt. Der Ansatz wird 1 h bei RT und danach 30 min bei 50°C gerührt. Man saugt vom Oxidationsmittel ab, wäscht den Filterrückstand mit Dioxan (3 x je 50 ml) und engt das Filtrat am Rotationsverdampfer ein. Das erhaltene Rohmaterial wird an Kieselgel (Laufmittelgradient: Cyclohexan → Cyclohexan/Essigsäureethylester 3:1) chromatographisch gereinigt. Man erhält 6.50 g (76% d. Th.) der Titelverbindung.
LC-MS (Methode 5): Rₜ = 2.14 min; MS (ESIpos): m/z = 209 [M+H]⁺
¹H-NMR (400 MHz, DMSO-d₆): δ = 7.50 (dd, 1H), 7.62 (dd, 1H), 7.69 (m, 2H), 7.90 (d, 1H), 8.12 (d, 1H), 8.39 (d, 1H), 10.5 (s, 1H).

### Beispiel 17A

### 3-Oxo-2-[(9-oxo-9H-fluoren-4-yl)methylen]butannitril

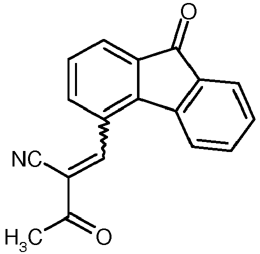

Die Verbindung aus Beispiel 10A (5.21 g, 25.0 mmol) wird in 180 ml Dichlormethan vorgelegt, und die Verbindung aus Beispiel 16A (2.89 g, 27.5 mmol), Essigsäure (1.72 ml, 30.0 mmol) und Piperidin (0.25 ml, 2.50 mmol) werden zugegeben. Der Ansatz wird 4 h lang am Wasserabscheider in der Siedehitze gerührt. Nach dem Abkühlen auf RT wird mit 30 ml Dichlormethan verdünnt, mit Wasser (2 x 50 ml) gewaschen, die organische Phase über Natriumsulfat getrocknet und das Lösungsmittel am Rotationsverdampfer entfernt. Das erhaltene Rohprodukt wird über Kieselgel-60 mit Dichlormethan als Laufmittel chromatographisch gereinigt. Man erhält nach Vereinigen der Produktfraktionen und Entfernen des Lösungsmittels 5.40 g (79% d. Th.) der Titelverbindung als *E*/*Z*-Isomerengemisch.
LC-MS (Methode 5): Rt = 2.24 min; MS (ESIpos): m/z = 274 [M+H]⁺
¹H-NMR (400 MHz, DMSO-d₆): δ = 2.64 (s, 3H), 7.49 (t, 1H), 7.59 (t, 1H), 7.64 (d, 1H), 7.69 (d, 1H), 7.73 (d, 1H), 7.81 (d, 1H), 7.90 (d, 1H), 8.88 (s, 1H).

### Beispiel 18A

### 6-Ethoxypyrimidin-2,4-diamin

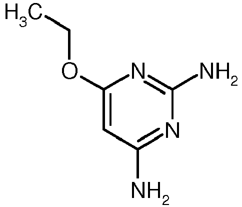

Zu einer kräftig gerührten Lösung von 500 mg (3.96 mmol) 2,6-Diaminopyrimidin-4-ol in 10 ml DMF werden unter einer Argonatmosphäre 174 mg Natriumhydrid (60% in Mineralöl, 4.36 mmol) portionsweise zugegeben. Nach 30 min werden 700 µl (5.15 mmol) Ethyl trifluormethansulfonat zugetropft und die Lösung für 20 min weitergerührt. Die Reaktionsmischung wird dann mit Methanol (1 ml) versetzt und direkt durch präparative HPLC aufgereinigt. Nach Vereinigung der Produktfraktionen und Entfernen des Lösungsmittels erhält man 370 mg (64% d. Th.) der Titelverbindung als weißen Feststoff.
LC-MS (Methode 5): Rₜ = 2.24 min; MS (ESIpos): m/z = 155 [M+H]⁺
¹H-NMR (400 MHz, DMSO-d₆): δ = 1.21 (t, 3H), 4.12 (q, 2H), 5.00 (s, 1H), 5.87 (s, 2H), 6.01 (s, 2H).

### Beispiel 19A

### 6-Acetyl-7-methyl-5-(2-methyl-4-oxo-4H-chromen-8-yl)-5,8-dihydropyrido[2,3-d]pyrimidin-4(3H)-on

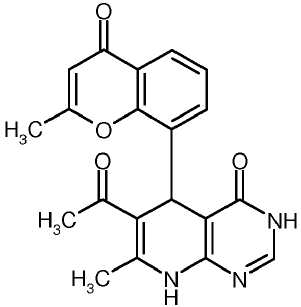

500 mg (1.85 mmol) 3-[(2-Methyl-4-oxo-4*H*-chromen-8-yl)methylen]pentan-2,4-dion werden mit 308 mg (2.77 mmol) 6-Aminopyrimidin-4(3*H*)-on versetzt, in 10 ml Isopropanol gelöst und unter Argon 2 Tage lang unter Rückfluss erhitzt. Das Gemisch wird danach eingeengt und der Rückstand aus Methanol umkristallisiert. Man erhält 341 mg (51% d. Th.) der Titelverbindung als gelben Feststoff.
LC-MS (Methode 1): Rₜ = 1.08 min; [M+H]⁺ = 364
¹H-NMR (300 MHz, DMSO-d₆): δ = 2.12 (s, 3H), 2.30 (s, 3H), 2.36 (s, 3H), 5.49 (s, 1H), 6.18 (s, 1H), 7.33 (t, 1H), 7.66 (dd, 1H), 7.80 (dd, 1H), 7.91 (s, 1H), 9.35 (s, 1H), 11.95 (br. s, 1H).

### Beispiel 20A

### 6-Isopropoxypyrimidin-2,4-diamin

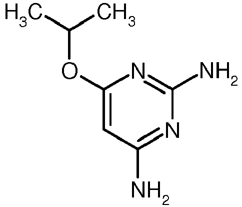

Zu einer kräftig gerührten Lösung von 1.8 g (14.27 mmol) 2,6-Diaminopyrimidin-4-ol in 20 ml DMF werden unter einer Argonatmosphäre 634 mg Natriumhydrid (60% in Mineralöl, 17.12 mmol) portionsweise zugegeben. Nach 30 min werden 1.6 ml (17.12 mmol) Isopropylbromid zugetropft und die Lösung für 12 h bei 40°C gerührt. Die Reaktionsmischung wird dann mit Methanol (1 ml) versetzt und direkt durch präparative HPLC aufgereinigt. Nach Vereinigung der Produktfraktionen und Entfernen des Lösungsmittels erhält man 250 mg (12% d. Th.) der Titelverbindung als weißen Feststoff.
LC-MS (Methode 5): Rₜ = 2.31 min; MS (ESIpos): m/z = 169 [M+H]⁺
¹H-NMR (400 MHz, DMSO-d₆): δ = 1.31 (d, 6H), 5.22 (s, 1H), 5.25 (m, 1H), 6.19 (s, 2H), 7.25 (s, 2H).

### Beispiel 21A

### 8-(6-Acetyl-2-amino-4-hydroxy-7-methyl-5,8-dihydropyrido[2,3-d]pyrimidin-5-yl)-2-methyl-4H-chromen-4-on

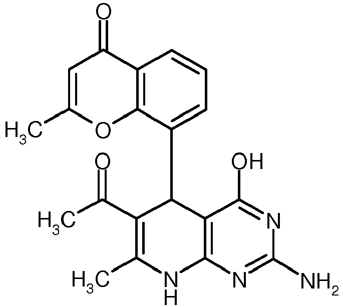

500 mg (1.85 mmol) 3-[(2-Methyl-4-oxo-4*H*-chromen-8-yl)methylen]pentan-2,4-dion werden mit 349 mg (2.77 mmol) 2,6-Diaminopyrimidin-4-ol versetzt, in 10 ml Isopropanol gelöst und unter Argon 2 Tage lang unter Rückfluss erhitzt. Es wird filtriert und der zurückbleibende Feststoff mit Isopropanol gewaschen. Man erhält 660 mg (94% d. Th.) der Titelverbindung als weißen Feststoff.
LC-MS (Methode 1): Rₜ = 1.03 min; [M+H]⁺ = 379
¹H-NMR (300 MHz, DMSO-d₆): δ = 2.08 (s, 3H), 2.27 (s, 3H), 2.36 (s, 3H), 5.34 (s, 1H), 6.17 (s, 1H), 6.28 (s, 2H), 7.30 (t, 1H), 7.62 (dd, 1H), 7.78 (dd, 1H), 9.25 (s, 1H), 10.22 (s, 1H).

### Beispiel 22A

### Ethyl 2-(4-cyano-2-methoxybenzyliden)-3-oxobutanoat

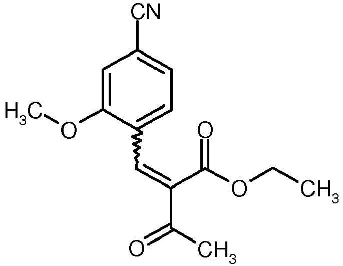

3 g (18.61 mmol) 4-Formyl-3-methoxybenzonitril, 2.6 ml (20.47 mmol) Ethyl 3-oxobutanoat, 1.33 ml (23.26 mmol) Essigsäure und 0.18 ml (1.85 mmol) Piperidin werden in 70 ml trockenem Dichlormethan 24 h lang am Wasserabscheider unter Rückfluss gerührt. Nach dem Abkühlen wird die Reaktionslösung nacheinander mit gesättigter Natriumhydrogencarbonat-Lösung und gesättigter Natriumchlorid-Lösung gewaschen. Die organische Phase wird über Magnesiumsulfat getrocknet und eingeengt. Der Rückstand wird aus Diethylether umkristallisiert. Man erhält 5.01 g (98% d. Th.) der Titelverbindung als *E*/*Z*-Isomerengemisch in Form eines leicht gelben Feststoffs.
¹H-NMR (300 MHz, DMSO-d₆): δ = 1.11 (t, 2H), 1.26 (t, 1H), 2.31 (s, 1H), 2.42 (s, 2H), 3.88 (s, 1H), 3.90 (s, 2H), 4.16 (q, 1.3H), 4.25 (q, 0.7H), 7.38 (d, 0.35H), 7.42 (d, 0.75H), 7.45 (dd, 0.35H), 7.49 (dd, 0.65H), 7.60 (d, 0.35H), 7.62 (d, 0.65H), 7.67 (s, 0.35H), 7.80 (s, 0.65H).

### Beispiel 23A

### Ethyl 2-(4-cyano-2-methoxybenzyliden)-4,4-diethoxy-3-oxobutanoat

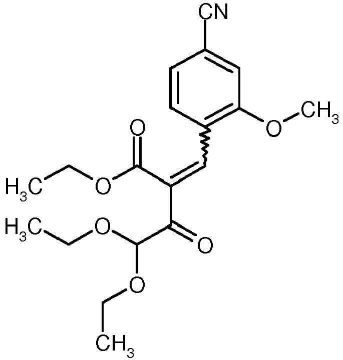

618.2 mg (2.83 mmol) Ethyl 4,4-diethoxy-3-oxobutanoat [Johnson et al., J. *Am*. *Chem*. *Soc.* 41, 812 (1919)], 500.0 mg (2.58 mmol) 4-Formyl-3-methoxybenzonitril, 232.0 mg (3.86 mmol) Essigsäure und 43.9 mg (0.51 mmol) Piperidin werden in 20 ml Dichlormethan gelöst und über Nacht an einem inversen Wasserabscheider unter Rückfluss erhitzt. Nach dem Abkühlen wird zweimal mit Wasser gewaschen und die organische Phase mit Magnesiumsulfat getrocknet. Das Lösungsmittel wird am Rotationsverdampfer entfernt und der Rückstand säulenchromatographisch aufgereinigt (Kieselgel, Laufmittel: Cyclohexan/Ethylacetat 4:1). Es werden 824 mg (77.0% d. Th.) der Titelverbindung als Gemisch der E/Z-Isomere erhalten.
LC-MS (Methode 4): Rₜ = 2.63 min und 2.69 min; [M-EtOH+H]⁺ (EIpos): m/z = 316.

### Beispiel 24A

### Ethyl 5-acetyl-4-(4-cyano-2-methoxyphenyl)-2-formyl-6-methyl-1,4-dihydropyridin-3-carboxylat

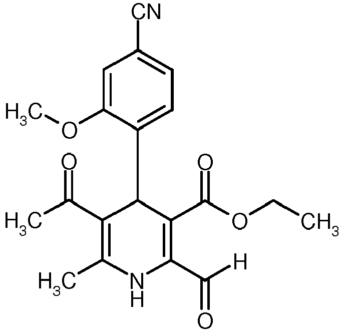

720 mg (1.992 mmol) Ethyl 2-(4-cyano-2-methoxybenzyliden)-4,4-diethoxy-3-oxobutanoat werden in 15 ml Isopropanol aufgenommen, mit 197.5 mg (4.84 mmol) 4-Amino-3-penten-2-on versetzt und über Nacht auf Rückflusstemperatur erhitzt. Der Ansatz wird danach eingeengt und der Rückstand mittels präparativer HPLC gereinigt (Eluent: Acetonitril/Wasser mit 0.1% Ameisensäure, Gradient 20:80 → 95:5). Es werden 250 mg (28.6% d. Th.) Ethyl 5-acetyl-4-(4-cyano-2-methoxyphenyl)-2-(diethoxymethyl)-6-methyl-1,4-dihydropyridin-3-carboxylat erhalten [LC-MS (Methode 11): Rt = 2.48 min; [M+H]⁺ (EIpos): m/z = 443].

250 mg (0.565 mmol) des so erhaltenen Dihydropyridin-Acetals werden in 7 ml Aceton aufgenommen und mit 0.38 ml 6 N Salzsäure versetzt. Es wird bei Raumtemperatur gerührt, bis vollständiger Umsatz detektiert ist (ca. 2 h, DC-Kontrolle). Das Reaktionsgemisch wird mit Natriumhydrogencarbonat-Lösung neutralisiert und das Aceton am Rotationsverdampfer entfernt. Es wird dreimal mit Ethylacetat extrahiert und die vereinigten organischen Phasen mit Natriumhydrogencarbonat-Lösung gewaschen. Nach dem Trocknen mit Magnesiumsulfat wird das Lösungsmittel unter vermindertem Druck abdestilliert und der Rückstand im Hochvakuum getrocknet. Es werden 824 mg (77.0% d. Th.) der Titelverbindung erhalten.
LC-MS (Methode 1): Rₜ = 2.00 min; [M+H]⁺ (EIpos): m/z = 369
¹H-NMR (300 MHz, DMSO-d₆): δ = 1.21 (t, 3H), 2.18 (s, 3H), 2.33 (s, 3H), 3.82 (s, 3H), 4.17 (m, 2H), 5.37 (s, 1H), 7.12 (d, 1H), 7.33 (d, 1H), 7.43 (s, 1H), 9.06 (s, 1H), 10.90 (br. s, 1H).

### Beispiel 25A

### 4-(3-Acetyl-2-methyl-5-oxo-1,4,5,6-tetrahydropyrido[2,3-d]pyridazin-4-yl)-3-methoxybenzonitril

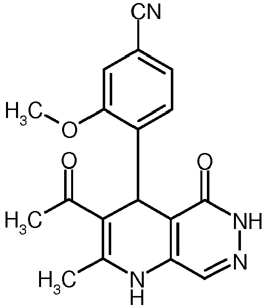

208 mg (0.565 mmol) Ethyl 5-acetyl-4-(4-cyano-2-methoxyphenyl)-2-formyl-6-methyl-1,4-di-hydropyridin-3-carboxylat und 41.5 mg (0.830 mmol) Hydrazin-Hydrat werden in 6.6 ml Ethanol/ Essigsäure (10:1) aufgenommen und 5 h bei 100°C umgesetzt. Die flüchtigen Komponenten werden am Rotationsverdampfer entfernt und der Rückstand in Acetonitril aufgenommen. Das ausfallende Produkt wird abfiltriert und im Hochvakuum getrocknet. Es werden 66 mg (34.7% d. Th.) der Titelverbindung erhalten.
LC-MS (Methode 4): Rₜ = 1.49 min; [M+H]⁺ (EIpos): m/z = 337
¹H-NMR (300 MHz, DMSO-d₆): δ = 2.14 (s, 3H), 2.19 (s, 3H), 3.81 (s, 3H), 5.36 (s, 1H), 7.20 (d, 1H), 7.31 (dd, 1H), 7.42 (d, 1H), 7.64 (s, 1H), 9.51 (s, 1H), 12.47 (s, 1H).

### Beispiel 26A

### Ethyl 4-(4-cyano-2-methoxyphenyl)-2-methyl-5-oxo-1,4,5,6-tetrahydropyrido[2,3-d]pyridazin-3-carboxylat

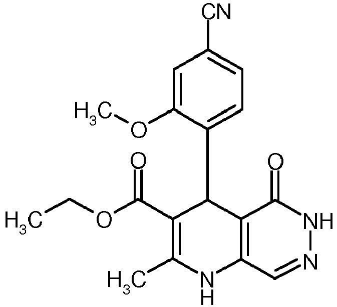

Analog zur Darstellung von Beispiel 24A ist Diethyl 4-(4-cyano-2-methoxyphenyl)-2-formyl-6-methyl-1,4-dihydropyridin-3,5-dicarboxylat ausgehend von Ethyl 3-aminobut-2-enoat und Ethyl 2-(4-cyano-2-methoxybenzyliden)-4,4-diethoxy-3-oxobutanoat (Beispiel 23A) erhältlich [vgl. auch Satoh et al., Chem. Pharm. Bull. 39, 3189-3201 (1991)].

115.5 mg (0.290 mmol) des so hergestellten Formyl-Dihydropyridins und 21.3 mg (0.426 mmol) Hydrazin-Hydrat werden in 6.6 ml Ethanol/Essigsäure (10:1) aufgenommen und 5 h bei 100°C umgesetzt. Die flüchtigen Komponenten werden am Rotationsverdampfer entfernt und der Rückstand mittels Säulenchromatographie aufgereinigt (Biotage-Kartusche 40S, Eluent: Ethylacetat). Die Produktfraktionen werden eingeengt, der Rückstand mit Dichlormethan verrührt, das ausfallende Produkt abfiltriert und die gelben Kristalle im Hochvakuum getrocknet. Es werden 67 mg (63.0% d. Th.) der Titelverbindung erhalten.
LC-MS (Methode 1): Rₜ = 1.57 min; [M+H]⁺ (EIpos): m/z = 367
¹H-NMR (300 MHz, DMSO-d₆): δ = 1.09 (t, 3H), 2.27 (s, 3H), 3.76 (s, 3H), 3.92 (m, 2H), 5.23 (s, 1H), 7.29 (s, 2H), 7.37 (s, 1H), 7.59 (s, 1H), 9.51 (s, 1H), 12.40 (s, 1H).

### Ausführungsbeispiele:

### Beispiel 1

### 8-(6-Acetyl-4-ethoxy-7-methyl-5,8-dihydropyrido[2,3-d]pyrimidin-5-yl)-2-methyl-4H-chromen-4-on

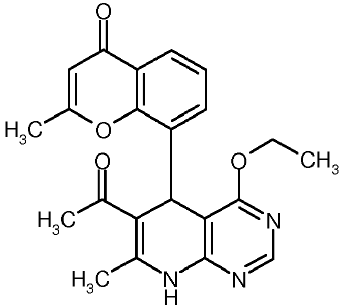

140 mg (0.38 mmol) 6-Acetyl-7-methyl-5-(2-methyl-4-oxo-4*H*-chromen-8-yl)-5,8-dihydropyrido-[2,3-d]pyrimidin-4(3H)-on werden unter Argonatmosphäre in Dichlormethan (7 ml) suspendiert, mit 219 mg (1.15 mmol) Triethyloxoniumtetrafluoroborat versetzt und 12 h bei RT gerührt. Die Reaktionsmischung wird dann mit Methanol versetzt und eingeengt. Man reinigt den Rückstand durch präparative HPLC und erhält 9 mg (6% d. Th.) der Titelverbindung als weißen Feststoff.
LC-MS (Methode 1): Rₜ = 1.62 min; [M+H]⁺ = 392
¹H-NMR (300 MHz, DMSO-d₆): δ = 1.05 (t, 3H), 2.14 (s, 3H), 2.35 (s, 3H), 2.39 (s, 3H), 4.15 (m, 2H), 5.65 (s, 1H), 6.22 (s, 1H), 7.34 (t, 1H), 7.66 (dd, 1H), 7.82 (dd, 1H), 8.25 (s, 1H), 10.06 (s, 1H).

### Beispiel 2

### 6-Acetyl-2-amino-7-methyl-5-(2-methyl-4-oxo-4H-chromen-8-yl)-5,8-dihydropyrido[2,3-d]pyrimidin-4-yl trifluormethansulfonat

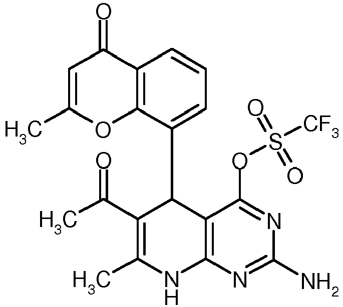

200 mg (0.52 mmol) 8-(6-Acetyl-2-amino-4-hydroxy-7-methyl-5,8-dihydropyrido[2,3-d]pyrimidin-5-yl)-2-methyl-4*H*-chromen-4-on werden in 5 ml Pyridin vorgelegt, mit 188 µl (1.057 mmol) Trifluormethansulfonsäureanhydrid versetzt und 30 min bei RT gerührt. Der Ansatz wird danach eingeengt und der Rückstand mittels präparativer HPLC gereinigt (Eluent: Acetonitril/Wasser mit 0.1% Ameisensäure, Gradient 20:80 → 95:5). Es werden 95 mg (35% d. Th.) der Titelverbindung als hellgelber Feststoff erhalten.
LC-MS (Methode 1): Rₜ = 1.85 min; MS (EIpos): m/z = 510 [M+H]⁺
¹H-NMR (300 MHz, DMSO-d₆): δ = 2.16 (s, 3H), 2.32 (s, 3H), 2.34 (s, 3H), 5.52 (s, 1H), 6.20 (s, 1H), 6.95 (s, 2H), 7.36 (t, 1H), 7.60 (dd, 1H), 7.84 (dd, 1H), 10.18 (s, 1H).

### Beispiel 3

### 6-Acetyl-7-methyl-5-(2-methyl-4-oxo-4H-chromen-8-yl)-5,8-dihydropyrido[2,3-d]pyrimidin-4-yl trifluormethansulfonat

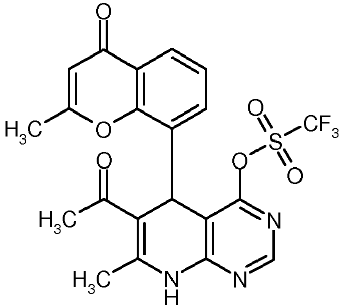

50 mg (0.13 mmol) 6-Acetyl-7-methyl-5-(2-methyl-4-oxo-4*H*-chromen-8-yl)-5,8-dihydropyrido-[2,3-d]pyrimidin-4(3H)-on werden in 2 ml Pyridin vorgelegt, mit 29 µl (0.165 mmol) Trifluormethansulfonsäureanhydrid versetzt und 30 min bei RT gerührt. Der Ansatz wird danach eingeengt und der Rückstand mittels präparativer HPLC gereinigt (Eluent: Acetonitril/Wasser mit 0.1% Ameisensäure, Gradient 20:80 → 95:5). Es werden 31 mg (45% d. Th.) der Titelverbindung als hellgelber Feststoff erhalten.
LC-MS (Methode 2): Rₜ = 2.31 min; MS (EIpos): m/z = 496 [M+H]⁺
¹H-NMR (300 MHz, DMSO-d₆): δ = 2.21 (s, 3H), 2.33 (s, 3H), 2.36 (s, 3H), 5.79 (s, 1H), 6.21 (s, 1H), 7.38 (t, 1H), 7.68 (dd, 1H), 7.88 (dd, 1H), 8.52 (s, 1H), 10.79 (s, 1H).

### Beispiel 4

### 8-(6-Acetyl-2-amino-4-ethoxy-7-methyl-5,8-dihydropyrido[2,3-d]pyrimidin-5-yl)-2-methyl-4H-chromen-4-on

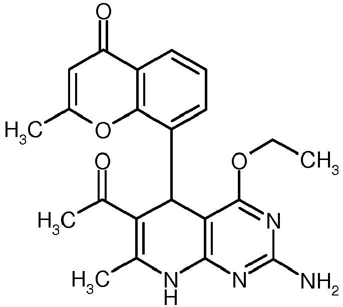

270 mg (1 mmol) 3-[(2-Methyl-4-oxo-4*H*-chromen-8-yl)methylen]pentan-2,4-dion und 170 mg (1.1 mmol) 6-Ethoxypyrimidin-2,4-diamin werden in 5 ml Isopropanol gelöst und unter Argon 2 Tage lang unter Rückfluss erhitzt. Das Gemisch wird danach eingeengt und der Rückstand mittels präparativer HPLC gereinigt. Man erhält 230 mg (56% d. Th.) der Titelverbindung als gelben Feststoff.
LC-MS (Methode 3): Rₜ = 1.68 min; [M+H]⁺ = 407
¹H-NMR (400 MHz, DMSO-d₆): δ = 1.00 (t, 3H), 2.09 (s, 3H), 2.31 (s, 3H), 2.39 (s, 3H), 4.04 (m, 2H), 5.48 (s, 1H), 6.18 (s, 2H), 6.21 (s, 1H), 7.32 (t, 1H), 7.59 (dd, 1H), 7.79 (dd, 1H), 9.49 (s, 1H).

### Beispiel 5

### 8-(6-Acetyl-2-amino-4-isopropoxy-7-methyl-5,8-dihydropyrido[2,3-d]pyrimidin-5-yl)-2-methyl-4H-chromen-4-on

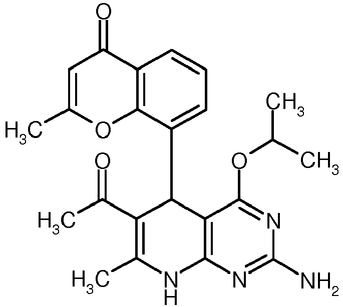

100 mg (0.37 mmol) 3-[(2-Methyl-4-oxo-4*H*-chromen-8-yl)methylen]pentan-2,4-dion und 62 mg (0.37 mmol) 6-Isopropoxypyrimidin-2,4-diamin werden in 5 ml Isopropanol gelöst und unter Argon 2 Tage lang unter Rückfluss erhitzt. Das Gemisch wird filtriert und der zurückbleibende Feststoff mit Isopropanol gewaschen. Man erhält 80 mg (51% d. Th.) der Titelverbindung als weißen Feststoff.
LC-MS (Methode 1): Rₜ = 1.63 min; [M+H]⁺ = 421
¹H-NMR (400 MHz, DMSO-d₆): δ = 0.66 (t, 3H), 1.19 (t, 3H), 2.08 (s, 3H), 2.30 (s, 3H), 2.40 (s, 3H), 5.01 (m, 1H), 5.44 (s, 1H), 6.15 (s, 2H), 6.22 (s, 1H), 7.32 (t, 1H), 7.58 (dd, 1H), 7.78 (dd, 1H), 9.46 (s, 1H).

### Beispiel 6

### 2-Amino-4-isopropoxy-7-methyl-5-(9-oxo-9H-fluoren-4-yl)-5,8-dihydropyrido[2,3-d]pyrimidin-6-carbonitril

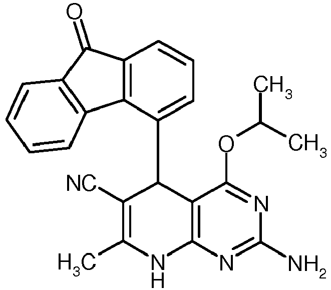

81 mg (0.29 mmol) 3-Oxo-2-[(9-oxo-9*H*-fluoren-4-yl)methylen]butannitril werden mit 50 mg (0.29 mmol) 6-Isopropoxypyrimidin-2,4-diamin in 5 ml Isopropanol gelöst und unter Argon 6 h lang unter Rückfluss erhitzt. Die Suspension wird nach dem Abkühlen abgesaugt und der zurückbleibende Feststoff mit Isopropanol gewaschen. Man erhält 74 mg (59% d. Th.) der Titelverbindung als weißen Feststoff.
LC-MS (Methode 8): Rt = 4.29 min; [M+H]⁺ = 424
¹H-NMR (400 MHz, CDCl₃): δ = 0.53 (d, 3H), 0.92 (d, 3H), 2.22 (s, 3H), 4.68 (s, 2H), 5.01 (m, 1H), 5.57 (s, 1H), 6.49 (s, 1H), 7.20 (t, 1H), 7.31 (t, 2H), 7.51 (t, 1H), 7.55 (d, 1H), 7.72 (d, 1H), 7.98 (d, 1H).

### Beispiel 7

### Ethyl 4-amino-5-(4-cyano-2-methoxyphenyl)-7-methyl-2-(methylthio)-5,8-dihydropyrido[2,3-d]-pyrimidin-6-carboxylat

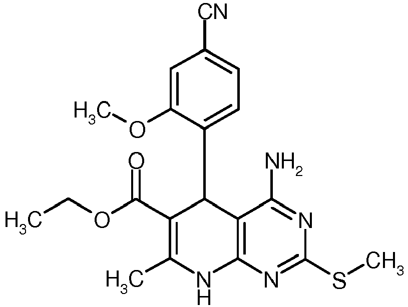

740 mg (2.70 mmol) Ethyl 2-(4-cyano-2-methoxybenzyliden)-3-oxobutanoat und 422 mg (2.70 mmol) 2-(Methylthio)-pyrimidin-4,6-diamin werden in 5 ml Isopropanol gelöst und unter Argon 12 h lang unter Rückfluss erhitzt. Das Gemisch wird filtriert und der zurückbleibende Feststoff mit Isopropanol gewaschen. Man erhält 395 mg (35% d. Th.) der Titelverbindung als weißen Feststoff.
LC-MS (Methode 1): Rₜ = 2.06 min; [M+H]⁺ = 412
¹H-NMR (400 MHz, DMSO-d₆): δ = 1.02 (t, 3H), 2.33 (s, 3H), 2.36 (s, 3H), 3.86 (q, 2H), 3.90 (s, 3H), 5.19 (s, 1H), 6.27 (s, 2H), 7.36 (s, 2H), 7.47 (s, 1H), 7.92 (dd, 1H), 9.58 (s, 1H).

### Beispiel 8

### 4-(3-Acetyl-5-ethoxy-2-methyl-1,4-dihydropyrido[2,3-d]pyridazin-4-yl)-3-methoxybenzonitril

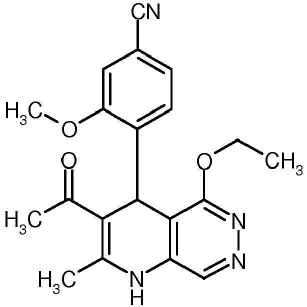

65 mg (0.193 mmol) 4-(3-Acetyl-2-methyl-5-oxo-1,4,5,6-tetrahydropyrido[2,3-d]pyridazin-4-yl)-3-methoxybenzonitril werden unter Argonatmosphäre mit 4 ml abs. Dichlormethan sowie 73.4 mg (0.386 mmol) Triethyloxoniumtetrafluoroborat versetzt. Nach einer zweistündigen Reaktionszeit bei Raumtemperatur (Reaktionskontrolle mittels HPLC) verbleibt der Umsatz unvollständig. Es werden zwei weitere Äquivalente Triethyloxoniumtetrafluoroborat hinzugefügt. Nach einer weiteren Reaktionszeit von 3 h wird der Ansatz mit 5 ml Methanol sowie 0.5 ml Wasser versetzt und nochmals für 2 h gerührt. Danach wird mit 20 ml Wasser verdünnt und dreimal mit Dichlormethan extrahiert. Die vereinigten organischen Phasen werden mit Natriumsulfat getrocknet und das Lösungsmittel am Rotationsverdampfer entfernt. Der Rückstand wird mittels präparativer HPLC gereinigt (Eluent: Acetonitril/Wasser mit 0.1% Ameisensäure, Gradient 20:80 → 95:5). Es werden 8 mg (11.3% d. Th.) der Titelverbindung erhalten.
LC-MS (Methode 1): Rₜ = 1.68 min; [M+H]⁺ (EIpos): m/z = 365
¹H-NMR (300 MHz, DMSO-d₆): δ = 1.20 (t, 3H), 2.11 (s, 3H), 2.28 (s, 3H), 3.84 (s, 3H), 4.27 (m, 2H), 5.47 (s, 1H), 7.26 (d, 1H), 7.31 (dd, 1H), 7.41 (d, 1H), 8.48 (s, 1H), 9.66 (s, 1H).

### Beispiel 9

### Ethyl 4-(4-cyano-2-methoxyphenyl)-5-ethoxy-2-methyl-1,4-dihydropyrido[2,3-d]pyridazin-3-carboxylat

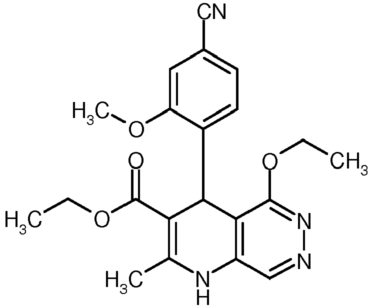

50 mg (0.136 mmol) Ethyl4-(4-cyano-2-methoxyphenyl)-2-methyl-5-oxo-1,4,5,6-tetrahydropyri-do[2,3-d]pyridazin-3-carboxylat werden unter Argonatmosphäre mit 5 ml abs. Dichlormethan sowie 51.8 mg (0.273 mmol) Triethyloxoniumtetrafluoroborat versetzt. Nach einer zweistündigen Reaktionszeit bei Raumtemperatur wird der Ansatz mit 5 ml Methanol sowie 0.5 ml Wasser versetzt und nochmals für 1 h gerührt. Danach wird mit 20 ml Wasser verdünnt und dreimal mit Dichlormethan extrahiert. Die vereinigten organischen Phasen werden mit Magnesiumsulfat getrocknet und das Lösungsmittel am Rotationsverdampfer entfernt. Der Rückstand wird mittels präparativer HPLC gereinigt (Eluent: Acetonitril/Wasser mit 0.1% Ameisensäure, Gradient 20:80 → 95:5). Es werden 15 mg (27.8% d. Th.) der Titelverbindung erhalten.
LC-MS (Methode 1): Rₜ = 1.97 min; [M+H]⁺ (EIpos): m/z = 395
¹H-NMR (300 MHz, DMSO-d₆): δ = 1.06 (t, 3H), 1.17 (t, 3H), 2.33 (s, 3H), 3.79 (s, 3H), 3.90 (q, 2H), 4.24 (m, 2H), 5.36 (s, 1H), 7.29 (m, 2H), 7.36 (s, 1H), 8.45 (s, 1H), 9.66 (s, 1H).

### B. Bewertung der pharmakologischen Wirksamkeit

### Abkürzungen:

- DMEM: Dulbecco's Modified Eagle Medium
- DNA: Desoxyribo Nucleic Acid
- FCS: Fetal Calf Serum
- HEPES: 4-(2-Hydroxyethyl)-1-piperazin-ethansulfonsäure
- PCR: Polymerase Chain Reaction
- Tris: Tris-(hydroxymethyl)-methylamin

Die vorteilhaften pharmakologischen Eigenschaften der erfindungsgemäßen Verbindungen können in folgenden Assays gezeigt werden:

### 1. Zellulärer in vitro-Test zur Bestimmung der inhibitorischen MR-Aktivität und MR-Selektivität gegenüber anderen Steroidhormon-Rezeptoren

Die Identifizierung von Antagonisten des humanen Mineralokorticoid-Rezeptors (MR) sowie die Quantifizierung der Wirksamkeit der hier beschriebenen Verbindungen erfolgt mit Hilfe einer rekombinanten Zelllinie. Die Zelle leitet sich ursprünglich von einer Ovarepithelzelle des Hamsters ab (Chinese Hamster Ovary, CHO K1, ATCC: American Type Culture Collection, VA 20108, USA).

In dieser CHO K1-Zelllinie wird ein etabliertes Chimärensystem verwendet, in dem die Liganden-Bindungsdomänen humaner Steroidhormon-Rezeptoren an die DNA-Bindungsdomäne des Hefe-Transkriptionsfaktors GAL4 fusioniert werden. Die so entstehenden GAL4-Steroidhormonrezeptor-Chimären werden in den CHO-Zellen mit einem Reporterkonstrukt co-transfiziert und stabil exprimiert.

### Klonierungen:

Zur Generierung der GAL4-Steroidhormonrezeptor-Chimären wird die GAL4-DNA-Bindungsdomäne (Aminosäuren 1-147) aus dem Vektor pFC2-dbd (Fa. Stratagene) mit den PCR-amplifizierten Liganden-Bindungsdomänen des Mineralokorticoid-Rezeptors (MR, Aminosäuren 734-985), des Glucokorticoid-Rezeptors (GR, Aminosäuren 443-777), des Progesteron-Rezeptors (PR, Aminosäuren 680-933) und des Androgen-Rezeptors (AR, Aminosäuren 667-919) in den Vektor pIRES2 (Fa. Clontech) kloniert. Das Reporterkonstrukt, welches fünf Kopien der GAL4-Bindestelle, vorgeschaltet vor einem Thymidinkinase-Promotor enthält, führt zur Expression der Firefly-Luciferase (*Photinus pyralis*) nach Aktivierung und Bindung der GAL4-Steroidhormonrezeptor-Chimären durch die jeweiligen spezifischen Agonisten Aldosteron (MR), Dexamethason (GR), Progesteron (PR) und Dihydrotestosteron (AR).

### Testablauf:

Die MR-, GR-, PR- und AR-Zellen werden am Tag vor dem Test in Medium (Optimem, 2.5% FCS, 2 mM Glutamin, 10 mM HEPES) in 96- (oder 384- bzw. 1536-) Loch-Mikrotiterplatten ausplattiert und in einem Zellinkubator (96% Luftfeuchtigkeit, 5% v/v CO₂, 37°C) gehalten. Am Testtag werden die zu prüfenden Substanzen in oben genanntem Medium aufgenommen und zu den Zellen hinzugegeben. Etwa 10 bis 30 Minuten nach Zugabe der Testsubstanzen werden die jeweiligen spezifischen Agonisten der Steroidhormon-Rezeptoren hinzugesetzt. Nach einer weiteren Inkubationszeit von 5 bis 6 Stunden wird die Luciferaseaktivität mit Hilfe einer Videokamera gemessen. Die gemessenen relativen Lichteinheiten ergeben in Abhängigkeit von der Substanzkonzentration eine sigmoide Stimulationskurve. Die Berechnung der IC₅₀-Werte erfolgt mit Hilfe des Computerprogramms GraphPad PRISM (Version 3.02).

Die erfindungsgemäßen Verbindungen weisen im MR-Test IC₅₀-Werte im Bereich von 20-250 nM auf.

### 2. In vitro-Test zur Bestimmung möglicher Bindungsaktivität am L-Typ Calcium-Kanal

Membranpräparationen des zerebralen Cortex von Wistar-Ratten dienen als Ausgangsmaterial für einen radioaktiven Bindungstest, der als Standardassay in der Literatur ausführlich beschrieben ist [Ehlert, F.J., Roeske, W.R., Itoga E., Yamamura, H.I., Life Sci. 30, 2191-2202 (1982); Gould, R.J., Murphy, K.M.M., Snyder, S.H., Proc. Natl. Acad. Sci. U.S.A. 79, 3656-3660] und von kommerziellen Anbietern (z.B. Fa. MDS Pharma Services) im Rahmen von Auftragsuntersuchungen verwendet wird. In diesem Bindungsassay werden Verdünnungsreihen der Testverbindungen in DMSO typischerweise für 90 Minuten bei 25°C in einem 50 mM TrisHCl-Puffer, pH 7.7, mit den Membranpräparationen und dem Tritium-markierten Liganden Nitrendipin (0.1 nM) inkubiert und die spezifische Bindung der Testverbindungen über Quantifizierung des spezifisch verdrängten, radioaktiv markierten Liganden bestimmt. IC₅₀-Werte werden über eine nicht-lineare Regressionsanalyse ermittelt.

In diesem L-Typ Calcium-Kanal-Bindungsassay wird für einen klassischen Calcium-Antagonisten vom Dihydropyridin-Typ, wie z.B. Nitrendipin, ein IC₅₀-Wert von 0.3 nM bestimmt, während untersuchte Beispiele der hier beschriebenen erfindungsgemäßen Verbindungen IC₅₀-Werte von ≥ 150 nM und somit eine mindestens um den Faktor 500 verminderte Affinität zum L-Typ Calcium-Kanal aufweisen. Verbindungen mit einer solch verringerten Residualbindungsaffinität zum L-Typ Calcium-Kanal zeigen in der Regel *in vivo* keine ausgeprägten hämodynamischen Effekte mehr, die über den L-Typ Calcium-Kanal vermittelt sind.

### 3. In vivo-Test zum Nachweis der kardiovaskulären Wirkung: Diurese-Untersuchungen an wachen Ratten in Stoffwechselkäfigen

Wistar-Ratten (250-350 g Körpergewicht) werden mit freiem Zugang zu Futter (Altromin) und Trinkwasser gehalten. Ab ca. 72 Stunden vor Versuchsbeginn erhalten die Tiere anstelle des normalen Futters ausschließlich Kochsalz-reduziertes Futter mit einem Gehalt von 0.02% Natriumchlorid (ssniff R/M-H, 10 mm mit 0.02% Na, S0602-E081, Fa. ssniff Spezialdiäten GmbH, D-59494 Soest). Während des Versuches werden die Tiere für ca. 24 Stunden einzeln in für Ratten dieser Gewichtsklasse geeigneten Stoffwechselkäfigen (Fa. Tecniplast Deutschland GmbH, D-82383 Hohenpeißenberg) mit freiem Zugang zu Kochsalz-reduziertem Futter und Trinkwasser gehalten. Am Versuchsbeginn wird den Tieren die zu prüfende Substanz in einem Volumen von 0.5 ml/kg Körpergewicht eines geeigneten Lösemittels mittels einer Schlundsonde in den Magen verabreicht. Als Kontrolle dienende Tiere erhalten nur Lösemittel. Kontrollen und Substanztestungen werden am selben Tag parallel durchgeführt. Kontrollgruppen und Substanz-Dosisgruppen bestehen aus jeweils 3 bis 6 Tieren. Während des Versuchs wird der von den Tieren ausgeschiedene Urin kontinuierlich in einem Auffangbehälter am Käfigboden gesammelt. Für jedes Tier wird gesondert das Urinvolumen pro Zeiteinheit bestimmt und die Konzentration der im Urin ausgeschiedenen Natrium- bzw. Kalium-Ionen mittels flammenphotometrischer Standardmethoden gemessen. Aus den Messwerten wird der Natrium/Kalium-Quotient als ein Maß für die Substanzwirkung berechnet. Die Messintervalle betragen typischerweise den Zeitraum bis zu 8 Stunden nach Versuchsbeginn (Tagintervall) und den Zeitraum von 8 bis 24 Stunden nach Versuchsbeginn (Nachtintervall). In einer abgewandelten Versuchsanordnung wird der Urin während des Tagintervalls im Abstand von zwei Stunden gesammelt und gemessen. Um eine hierfür ausreichende Urinmenge zu erhalten, wird den Tieren zu Versuchsbeginn und dann im Abstand von zwei Stunden per Schlundsonde eine definierte Menge Wasser zugeführt.

### 4. DOCA/Salz-Modell

Die Verabreichung von Desoxycorticosteronacetat (DOCA) in Kombination mit einer Hochsalzdiät und einseitiger Nierenentfernung induziert bei der Ratte einen Hypertonus, der durch relativ niedrige Reninspiegel charakterisiert ist. Als Folge dieser endokrinen Hypertonie (DOCA ist eine direkte Vorstufe von Aldosteron) kommt es in Abhängigkeit von der gewählten DOCA-Konzentration zu einer Hypertrophie des Herzens und weiteren Endorgan-Schäden, z.B. der Niere, die u.a. durch Proteinurie und Glomerulosklerose charakterisiert sind. In diesem Rattenmodell lassen sich somit Testsubstanzen auf vorhandene antihypertrophe und Endorgan-schützende Wirkung hin untersuchen.

Etwa 8 Wochen alte (Körpergewicht zwischen 250 und 300 Gramm), männliche Sprague Dawley (SD)-Ratten werden linksseitig uninephrektomiert. Dazu werden die Ratten mit 1.5-2%-igem Isofluran in einer Mischung aus 66% N₂O und 33% O₂ anästhesiert und die Niere über einen Flankenschnitt entfernt. Als spätere Kontrolltiere dienen sogenannte sham-operierte Tiere, denen keine Niere entfernt wird.

Uninephrektomierte SD-Ratten erhalten 1% Natriumchlorid im Trinkwasser und einmal wöchentlich eine subkutane Injektion von Desoxycorticosteronacetat (gelöst in Sesamöl; Fa. Sigma) zwischen die Schulterblätter gespritzt (Hochdosis: 100 mg/kg/Woche s.c.; Normaldosis: 30 mg/kg/ Woche s.c.).

Die Substanzen, die auf ihre protektive Wirkung *in vivo* untersucht werden sollen, werden per Gavage oder über das Futter (Fa. Ssniff) verabreicht. Die Tiere werden einen Tag vor Versuchsbeginn randomisiert und Gruppen mit gleicher Tierzahl, in der Regel n = 10, zugeordnet. Während des gesamten Versuchs steht den Tieren Trinkwasser und Futter *ad libitum* zur Verfügung. Die Substanzen werden einmal täglich 4-8 Wochen lang per Gavage oder per Futter verabreicht. Als Plazebogruppe dienen Tiere, die genauso behandelt werden, aber entweder nur das Lösungsmittel oder das Futter ohne Testsubstanz erhalten.

Die Wirkung der Testsubstanzen wird durch Messung hämodynamischer Parameter [Blutdruck, Herzfrequenz, Inotropie (dp/dt), Relaxationszeit (tau), maximaler linksventrikulärer Druck, linksventrikulärer enddiastolischer Druck (LVEDP)], Gewichtsbestimmung von Herz, Niere und Lunge, Messung der Proteinausscheidung sowie durch Messung der Genexpression von Biomarkern (z.B. ANP, Atrial Natriuretic Peptide, und BNP, Brain Natriuretic Peptide) mittels RT/TaqMan-PCR nach RNA-Isolation aus kardialem Gewebe bestimmt.

Die statistische Auswertung erfolgt mit Student's t-Test nach vorheriger Überprüfung der Varianzen auf Homogenität.

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel® (Xanthan gum der Fa. FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die Lösung wird steril filtriert und in sterile und pyrogenfreie Injektionsbehältnisse abgefüllt.

## Patentansprüche

1. Verbindung der Formel (I) 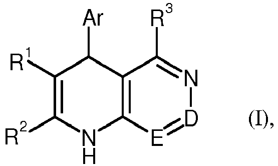 in welcher
D für C-R⁴ steht, worin
R⁴ Wasserstoff, Amino, Methoxy oder Methylthio bedeutet,
E für N steht,
Ar für eine Gruppe der Formel 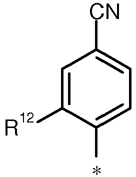 steht, worin
* die Verknüpfungsstelle
und
R¹² Ethyl, Methoxy oder Trifluormethoxy bedeutet,
R¹ für Cyano, Acetyl, Methoxycarbonyl oder Ethoxycarbonyl steht,
R² für Methyl oder Trifluormethyl steht
und
R³ für Amino, (C₁-C₃)-Alkoxy oder eine Gruppe der Formel -O-SO₂-R¹⁶ steht, worin
R¹⁶ (C₁-C₃)-Alkyl bedeutet,
sowie ihre Salze, Solvate und Solvate der Salze.

2. Verfahren zur Herstellung von Verbindungen der Formel (I-B) 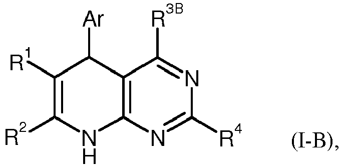 in welcher Ar, R¹, R² und R⁴ die in Anspruch 1 angegebenen Bedeutungen haben
und
R^{3B} für Amino, (C₁-C₃)-Alkoxy oder eine Gruppe der Formel -O-SO₂-R¹⁶ steht, worin
R¹⁶ (C₁-C₃)-Alkyl bedeutet,
**dadurch gekennzeichnet, dass** man eine Verbindung der Formel (II) 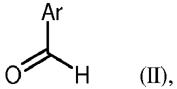 in welcher Ar die in Anspruch 1 angegebene Bedeutung hat,
mit einer Verbindung der Formel (XI) 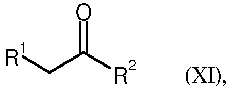 in welcher R¹ und R² die in Anspruch 1 angegebenen Bedeutungen haben,
zu einer Verbindung der Formel (XII) 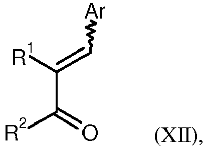 in welcher Ar, R¹ und R² jeweils die oben angegebenen Bedeutungen haben, kondensiert und diese anschließend entweder
[B-1] in einem inerten Lösungsmittel mit einer Verbindung der Formel (XIII) 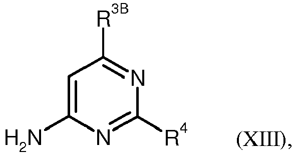 in welcher R^{3B} und R⁴ die oben bzw. in Anspruch 1 angegebenen Bedeutungen haben,
umsetzt
oder
[B-2] zunächst in einem inerten Lösungsmittel mit einer Verbindung der Formel (XIV) 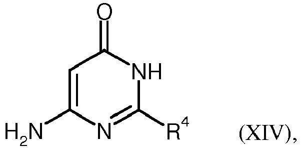 in welcher R⁴ die in Anspruch 1 angegebene Bedeutung hat, zu einer Verbindung der Formel (XV) 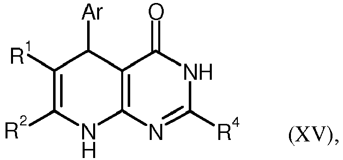 in welcher Ar, R¹, R² und R⁴ jeweils die oben angegebenen Bedeutungen haben,
umsetzt und diese dann in einem inerten Lösungsmittel gegebenenfalls in Gegenwart einer Base mit einer Verbindung der Formel (VIII) oder einem Trialkyloxonium-Salz der Formel (IX)
R¹⁷-O (VIII) 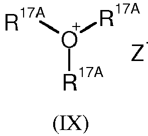
in welchen
R¹⁷ für (C₁-C₆)-Alkyl, das mit (C₃-C₇)-Cycloalkyl substituiert sein kann, oder für Trifluormethyl steht,
R^{17A} für Methyl oder Ethyl steht,
Q für eine Abgangsgruppe, wie beispielsweise Halogen, Mesylat, Tosylat oder Triflat, steht
und
Z⁻ für ein nicht-nukleophiles Anion, wie beispielsweise Tetrafluoroborat, steht,
zu Verbindungen der Formel (I-B1) 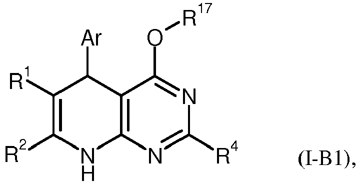 in welcher Ar, R¹, R², R⁴ und R¹⁷ jeweils die oben angegebenen Bedeutungen haben,
alkyliert
oder die Verbindungen der Formel (XV) in einem inerten Lösungsmittel in Gegenwart einer Base mit einer Verbindung der Formel (X) 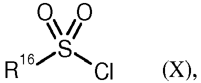 in welcher R¹⁶ die in Anspruch 1 angegebene Bedeutung hat,
zu Verbindungen der Formel (I-B2) 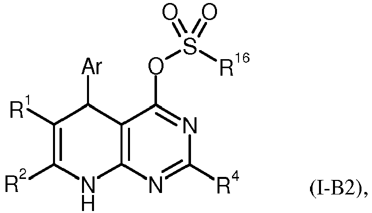 in welcher Ar, R¹, R², R⁴ und R¹⁶ jeweils die oben angegebenen Bedeutungen haben,
umsetzt
und gegebenenfalls die jeweils resultierenden Verbindungen der Formel (I-B), (I-B1) bzw. (I-B2) nach dem Fachmann bekannten Methoden in ihre Enantiomere und/oder Diastereomere trennt und/oder mit den entsprechenden (i) Lösungsmitteln und/oder (ii) Basen oder Säuren in ihre Solvate, Salze und/oder Solvate der Salze überführt.

3. Verbindung der Formel (I), wie in Anspruch 1 definiert, zur Behandlung und/oder Prophylaxe von Krankheiten.

4. Verwendung einer Verbindung der Formel (I), wie in Anspruch 1 definiert, zur Herstellung eines Arzneimittels zur Behandlung und/oder Prophylaxe von Aldosteronismus, Bluthochdruck, chronischer Herzinsuffizienz, den Folgen eines Myokardinfarkts, Leberzirrhose, Niereninsuffizienz und Hirnschlag.

5. Arzneimittel enthaltend eine Verbindung der Formel (I), wie in Anspruch 1 definiert, in Kombination mit einem inerten, nicht-toxischen, pharmazeutisch geeigneten Hilfsstoff.

6. Arzneimittel enthaltend eine Verbindung der Formel (I), wie in Anspruch 1 definiert, in Kombination mit einem oder mehreren weiteren Wirkstoffen ausgewählt aus der Gruppe bestehend aus ACE-Inhibitoren, Renin-Inhibitoren, Angiotensin 11-Rezeptor-Antagonisten, Beta-Blocker, Acetylsalicylsäure, Diuretika, Kalium-Supplements, Calcium-Antagonisten, Statine, Digitalis (Digoxin)-Derivate, Calcium-Sensitizer, Nitrate sowie Antithrombotika.

7. Arzneimittel nach Anspruch 5 oder 6 zur Behandlung und/oder Prophylaxe von Aldosteronismus, Bluthochdruck, chronischer Herzinsuffizienz, den Folgen eines Myokardinfarkts, Leberzirrhose, Niereninsuffizienz und Hirnschlag.

## Claims

1. Compound of the formula (I) 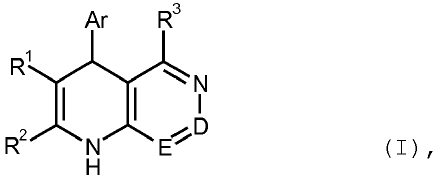 in which
D is C-R⁴ in which
R⁴ is hydrogen, amino, methoxy or methylthio,
E is N,
Ar is a group of the formula 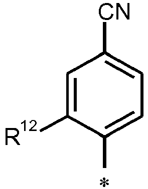 in which
* is the linkage point
and
R¹² is ethyl, methoxy or trifluoromethoxy,
R¹ is cyano, acetyl, methoxycarbonyl or ethoxycarbonyl,
R² is methyl or trifluoromethyl,
and
R³ is amino, (C₁-C₃) -alkoxy or a group of the formula -O-SO₂-R¹⁶ in which
R¹⁶ is (C₁-C₃) -alkyl,
and the salts, solvates and solvates of the salts thereof.

2. Process for preparing compounds of the formula (I-B) 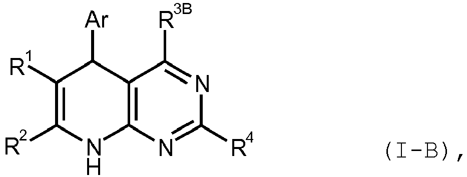 in which Ar, R¹, R² and R⁴ have the meanings indicated in Claim 1,
and
R^{3B} is amino, (C₁-C₃) -alkoxy or a group of the formula -O-SO₂-R¹⁶ in which
R¹⁶ is (C₁-C₃)-alkyl,
**characterized in that** a compound of the formula (II) 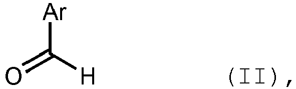 in which Ar has the meaning indicated in Claims 1, is condensed with a compound of the formula (XI) 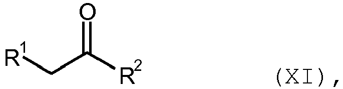 in which R¹ and R² have the meanings indicated in Claims 1,
to give a compound of the formula (XII) 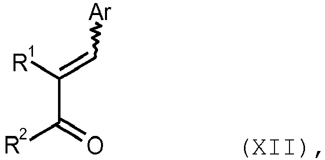 in which Ar, R¹ and R² each have the meanings indicated above,
and the latter is subsequently either
[B-1] reacted in an inert solvent with a compound of the formula (XIII) 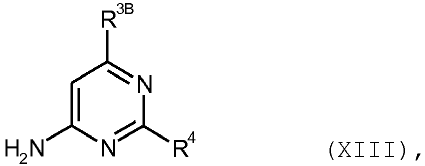 in which R^{3B} and R⁴ have the meanings indicated in Claims 1,
or
[B-2] is initially reacted in an inert solvent with a compound of the formula (XIV) 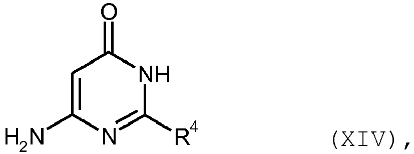 in which R⁴ has the meaning indicated in Claims 1,
to give a compound of the formula (XV) 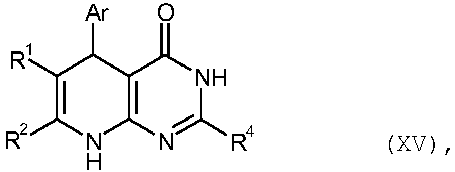 in which Ar, R¹, R² and R⁴ each have the meanings indicated above,
and the latter is then alkylated in an inert solvent, where appropriate in the presence of a base, with a compound of the formula (VIII) or a trialkyloxonium salt of the formula (IX)
R¹⁷-Q (VIII) 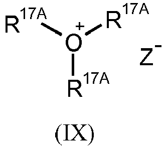
in which
R⁷ is (C₁-C₆)-alkyl which may be substituted by (C₃-C₇) -cycloalkyl, or is trifluoromethyl,
R^{17A} is methyl or ethyl,
Q is a leaving group such as, for example, halogen, mesylate, tosylate or triflate,
and
Z⁻ is a non-nucleophilic anion such as, for example, tetrafluoroborate,
to give compounds of the formula (I-B1) 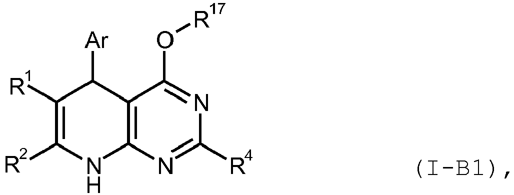 in which Ar, R¹, R², R⁴ and R¹⁷ each have the meanings indicated above,
or the compounds of the formula (XV) are reacted in an inert solvent in the presence of a base with a compound of the formula (X) 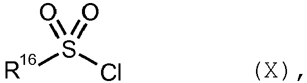 in which R¹⁶ has the meaning indicated in Claim 1,
to give compounds of the formula (I-B2) 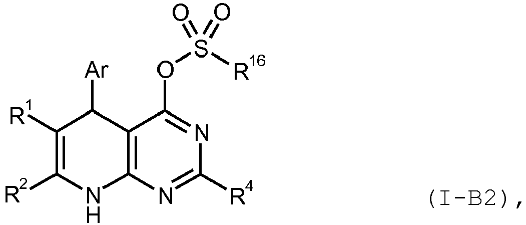 in which Ar, R¹, R², R⁴ and R¹⁶ each have the meanings indicated above,
and where appropriate the respective resulting compounds of the formula (I-B), (I-B1) or (I-B2) are separated by methods known to the skilled worker into their enantiomers and/or diastereomers, and/or converted with the appropriate (i) solvents and/or (ii) bases or acids into the solvates, salts and/or solvates of the salts thereof.

3. Compound of the formula (I) as defined in Claim 1 for the treatment and/or prophylaxis of diseases.

4. Use of a compound of the formula (I) as defined in Claim 1 for the manufacture of a medicament for the treatment and/or prophylaxis of aldosteronism, high blood pressure, chronic heart failure, the sequelae of a myocardial infarction, cirrhosis of the liver, renal failure and stroke.

5. Medicament comprising a compound of the formula (I) as defined in Claim 1 in combination with an inert, non-toxic, pharmaceutically suitable excipient.

6. Medicament comprising a compound of the formula (I) as defined in Claim 1 in combination with one or more further active ingredients selected from the group consisting of ACE inhibitors, renin inhibitors, angiotensin II receptor antagonists, beta-blockers, acetylsalicylic acid, diuretics, potassium supplements, calcium antagonists, statins, digitalis (digoxin) derivatives, calcium sensitizers, nitrates and antithrombotics.

7. Medicament according to Claim 5 or 6 for the treatment and/or prophylaxis of aldosteronism, high blood pressure, chronic heart failure, the sequelae of a myocardial infarction, cirrhosis of the liver, renal failure and stroke.

## Revendications

1. Composé de formule (I) 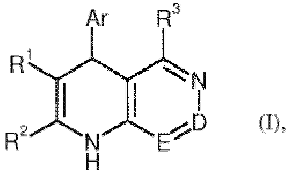 dans laquelle
D représente C-R⁴,
R⁴ signifiant hydrogène, amino, méthoxy ou méthylthio,
E représente N,
Ar représente un groupe de formule 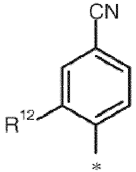 dans laquelle
* signifie l'emplacement de liaison
et
R¹² signifie éthyle, méthoxy ou trifluorométhoxy,
R¹ représente cyano, acétyle, méthoxycarbonyle ou éthoxycarbonyle,
R² représente méthyle ou trifluorométhyle,
et
R³ représente amino, alcoxy en (C₁-C₃) ou un groupe de formule -O-SO₂-R¹⁶,
R¹⁶ signifiant alkyle en (C₁-C₃),
ainsi que ses sels, solvates et solvates des sels.

2. Procédé de fabrication de composés de formule (I-B) 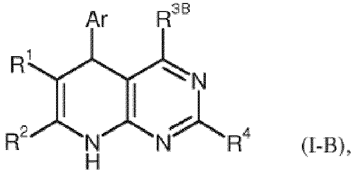
dans laquelle Ar, R¹, R² et R⁴ ont les significations indiquées dans la revendication 1,
et
R^{3B} représente amino, alcoxy en (C₁-C₃) ou un groupe de formule -O-SO₂-R¹⁶,
R¹⁶ signifiant alkyle en (C₁-C₃),
**caractérisé en ce qu'**un composé de formule (II) 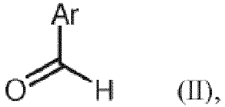 dans laquelle Ar a la signification indiquée dans la revendication 1,
est condensé avec un composé de formule (XI) 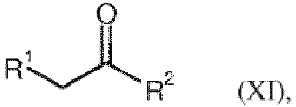 dans laquelle R¹ et R² ont les significations indiquées dans la revendication 1,
pour former un composé de formule (XII) 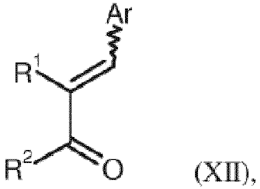 dans laquelle Ar, R¹ et R² ont chacun les significations indiquées précédemment,
puis celui-ci est
[B-1] mis en réaction dans un solvant inerte avec un composé de formule (XIII) 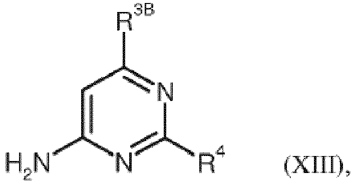 dans laquelle R^{3B} et R⁴ ont les significations indiquées précédemment ou dans la revendication 1,
ou
[B-2] tout d'abord mis en réaction dans un solvant inerte avec un composé de formule (XIV) 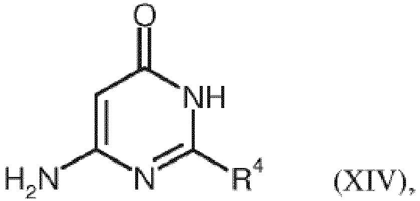 dans laquelle R⁴ a la signification indiquée dans la revendication 1,
pour former un composé de formule (XV) 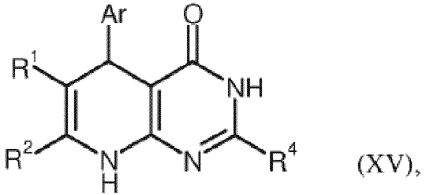 dans laquelle Ar, R¹, R² et R⁴ ont chacun les significations indiquées précédemment,
puis celui-ci est alkylé dans un solvant inerte, éventuellement en présence d'une base, avec un composé de formule (VIII) ou un sel de trialkyl-oxonium de formule (IX)
R¹⁷-Q **(VIII)** 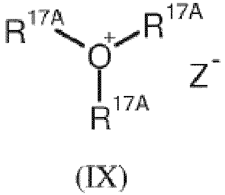
dans lesquelles
R¹⁷ représente alkyle en (C₁-C₆), qui peut être substitué avec cycloalkyle en (C₃-C₇), ou trifluorométhyle,
R^{17A} représente méthyle ou éthyle,
Q représente un groupe partant, tel que par exemple halogène, mésylate, tosylate ou triflate,
et
Z⁻ représente un anion non nucléophile, tel que par exemple tétrafluoroborate,
pour former des composés de formule (I-B1) 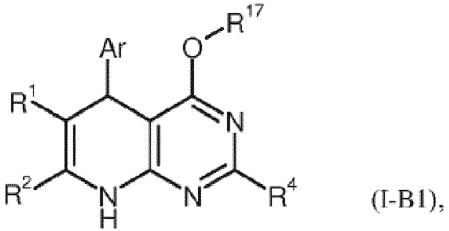 dans laquelle Ar, R¹, R², R⁴ et R¹⁷ ont chacun les significations indiquées précédemment,
ou les composés de formule (XV) sont mis en réaction dans un solvant inerte en présence d'une base avec un composé de formule (X) 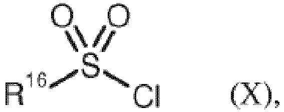 dans laquelle R¹⁶ a la signification indiquée dans la revendication 1,
pour former des composés de formule (I-B2) 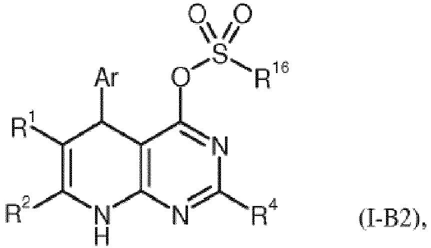 dans laquelle Ar, R¹, R², R⁴ et R¹⁶ ont chacun les significations indiquées précédemment,
et les composés résultants de formule (I-B), (I-B1) et (I-B2) respectifs sont éventuellement séparés par des méthodes connues de l'homme du métier en leurs énantiomères et/ou diastéréomères, et/ou transformés avec (i) les solvants et/ou (ii) les bases ou les acides appropriés en leurs solvates, sels et/ou solvates des sels.

3. Composé de formule (I), tel que défini dans la revendication 1, pour le traitement et/ou la prophylaxie de maladies.

4. Utilisation d'un composé de formule (I), tel que défini dans la revendication 1, pour la fabrication d'un médicament pour le traitement et/ou la prophylaxie de l'aldostéronisme, de l'hypertension, de l'insuffisance cardiaque chronique, des conséquences d'un infarctus du myocarde, de la cirrhose du foie, de l'insuffisance rénale et de l'attaque cérébrale.

5. Médicament contenant un composé de formule (I), tel que défini dans la revendication 1, en combinaison avec un adjuvant inerte, non toxique, pharmaceutiquement approprié.

6. Médicament contenant un composé de formule (I), tel que défini dans la revendication 1, en combinaison avec un ou plusieurs agents actifs supplémentaires choisis dans le groupe constitué par les inhibiteurs d'ACE, les inhibiteurs de rénine, les antagonistes du récepteur d'angiotensine II, les bêtabloquants, l'acide acétylsalicylique, les diurétiques, les suppléments de potassium, les antagonistes de calcium, les statines, les dérivés de digitalis (digoxine), les sensibilisateurs de calcium, les nitrates et les antithrombotiques.

7. Médicament selon la revendication 5 ou 6, pour le traitement et/ou la prophylaxie de l'aldostéronisme, de l'hypertension, de l'insuffisance cardiaque chronique, des conséquences d'un infarctus du myocarde, de la cirrhose du foie, de l'insuffisance rénale et de l'attaque cérébrale.
